# EUROPEAN PATENT SPECIFICATION

(11) **EP 1 761 649 B1**
(45) Date of publication and mention of the grant of the patent: **26.12.2007**
(21) Application number: 05764390.0
(22) Date of filing: 30.06.2005
(51) Int. Cl.: C12Q 1/68

(54) **HUMAN AUTISM PREDISPOSITION GENE ENCODING A TRANSCRIPTION FACTOR AND USES THEREOF**
EINEN TRANSKRIPTIONSFAKTOR KODIERENDES HUMANES PRÄDISPOSITIONSGEN FÜR AUTISMUS SOWIE DESSEN VERWENDUNGEN
GENE DE PREDISPOSITION A L'AUTISME HUMAIN CODANT POUR UN FACTEUR DE TRANSCRIPTION ET SES UTILISATIONS

(30) Priority: 01.07.2004 US 584130 P
(43) Date of publication of application: 14.03.2007
(73) Proprietor: Integragen, 91000 EVRY (FR)
(72) Inventor: PHILIPPI, Anne, 77310 St. Fargeau Ponthierry (FR); ROUSSEAU, Francis, 91600 Savigny sur Orge (FR); BROOKS, Peter, 75007 Paris (FR); HAGER, Jörg, 91540 Mennecy (FR)
(74) Representative: Chajmowicz, Marion
(86) International application number: PCT/IB2005/002319
(87) International publication number: WO 2006/003520

(56) References cited:
- WANG J ET AL: "The canonical Wnt pathway in early mammalian embryogenesis and stem cell maintenance/differentiation" CURRENT OPINION IN GENETICS & DEVELOPMENT, CURRENT BIOLOGY LTD, vol. 14, no. 5, October 2004 (2004-10), pages 533-539, XP004566207 ISSN: 0959-437X
- SEONG E ET AL: "Mouse models for psychiatric disorders" TRENDS IN GENETICS, ELSEVIER, AMSTERDAM, NL, vol. 18, no. 12, 1 December 2002 (2002-12-01), pages 643-650, XP004393480 ISSN: 0168-9525
- MONACO A P: "A genomewide screen for autism: Strong evidence for linkage to chromosomes 2q, 7q, and 16p: International molecular genetic study of autism consortium, (IMGSAC)" AMERICAN JOURNAL OF HUMAN GENETICS, AMERICAN SOCIETY OF HUMAN GENETICS, CHICAGO, IL, US, vol. 69, no. 3, 2001, pages 570-581, XP002347957 ISSN: 0002-9297
- PHILIPPE A ET AL: "Genome-wide scan for autism susceptibility genes" NUCLEIC ACIDS RESEARCH, OXFORD UNIVERSITY PRESS, SURREY, GB, vol. 8, no. 5, May 1999 (1999-05), pages 805-812, XP002323915 ISSN: 0305-1048
- TURNER M ET AL: "Genetic clues to the biological basis of autism" MOLECULAR MEDICINE TODAY, ELSEVIER, CAMBRIDGE, GB, vol. 6, no. 6, June 2000 (2000-06), pages 238-244, XP002323919 ISSN: 1357-4310
- SHASTRY B S: "Molecular genetics of autism spectrum disorders" JOURNAL OF HUMAN GENETICS, vol. 48, no. 10, 2003, pages 495-501, XP002315920

### SEQUENCE LISTING

<110> INTEGRAGEN
<120> HUMAN AUTISM PREDISPOSITION GENE ENCODING A TRANSCRIPTION FACTOR AND USES THEREOF
<130> B0298US
<150> US 60/584,130
   <151> 2004-07-01
<160> 37
<170> PatentIn version 3.1
<210> 1
   <211> 2373
   <212> DNA
   <213> Homo sapiens
<220>
   <221> CDS
   <222> (418)..(1362)
   <223>
<400> 1
   cccaggccca ccccacccag cacccctggc gcagggactg ctggaacctg gctgtgcgcg      60
   ctgtcgcttt aagacagact ctgccggcgc cgtccggagc cttagaaacc ggccccggat     120
   cgcgagccgg agccggagcc ggagccgggg ccggccgggc tgctgaggcc cgagcggcag     180
   gagcgcagcg cggagcgctg agccaggcgc ccagtcgcga gaagctgccg ccgcctctgc     240
   ccgcccggcg ccgcagcccc gggcggtcca tggggcgggc acggcgtcgc tgcaggcgcc     300
   ggcagccctg gagggcagcc gcttaggcgc tgcgctcttg tccccgcagg tcgcagccag     360
   ggcggcgggg cgcgcccagc cccggcccct ggagcgcccg ccgcggtccc cacctcc        417
   atg gac gcc ttc aag ggg ggc atg agc ctg gag cgg ctg ccg gag ggg       465
   Met Asp Ala Phe Lys Gly Gly Met Ser Leu Glu Arg Leu Pro Glu Gly
   1               5                   10                  15
   ctc cgg ccg ccg ccg ccg cca ccc cat gac atg ggg ccc gcc ttc cac      513
   Leu Arg Pro Pro Pro Pro Pro Pro His Asp Met Gly Pro Ala Phe His
               20                  25                  30
   ctg gcc cgg ccc gcc gac ccc cgc gag ccg ctc gag aac tcc gcc agc      561
   Leu Ala Arg Pro Ala Asp Pro Arg Glu Pro Leu Glu Asn Ser Ala Ser
           35                  40                  45
   gag tcg tct gac acg gag ctg cca gag aag gag cgc ggc ggg gaa ccc      609
   Glu Ser Ser Asp Thr Glu Leu Pro Glu Lys Glu Arg Gly Gly Glu Pro
       50                  55                  60
   aag ggg ccc gag gac agt ggt gcg gga ggc acg ggc tgc ggc ggc gca      657
   Lys Gly Pro Glu Asp Ser Gly Ala Gly Gly Thr Gly Cys Gly Gly Ala
   65                  70                  75                  80
   gac gac cca gcc aag aag aag aag cag cgg cgg caa cgt acg cac ttc      705
   Asp Asp Pro Ala Lys Lys Lys Lys Gln Arg Arg Gln Arg Thr His Phe
                   85                  90                  95
   aca agc cag cag ttg caa gag cta gag gcc acg ttc cag agg aac cgc      753
   Thr Ser Gln Gln Leu Gln Glu Leu Glu Ala Thr Phe Gln Arg Asn Arg
               100                 105                 110
   tac ccc gac atg agc atg agg gag gag atc gcc gtg tgg acc aac ctc      801
   Tyr Pro Asp Met Ser Met Arg Glu Glu Ile Ala Val Trp Thr Asn Leu
           115                 120                 125
   acc gag ccg cgc gtg cgg gtc tgg ttc aag aac cgg cga gcc aag tgg      849
   Thr Glu Pro Arg Val Arg Val Trp Phe Lys Asn Arg Arg Ala Lys Trp
       130                 135                 140
   cgt aag cgc gag cgt aac cag cag ctg gac ctg tgc aag ggt ggc tac      897
   Arg Lys Arg Glu Arg Asn Gln Gln Leu Asp Leu Cys Lys Gly Gly Tyr
   145                 150                 155                 160
   gtg ccg cag ttc agc ggc cta gtg cag ccc tac gag gac gtg tac gcc      945
   Val Pro Gln Phe Ser Gly Leu Val Gln Pro Tyr Glu Asp Val Tyr Ala
                   165                 170                 175
   gcc ggc tac tcc tac aac aac tgg gcc gcc aag agc ctg gcg cca gcg      993
   Ala Gly Tyr Ser Tyr Asn Asn Trp Ala Ala Lys Ser Leu Ala Pro Ala
               180                 185                 190
   ccg ctc tcc acc aag agc ttc acc ttc ttc aac tcc atg agc ccg ctg     1041
   Pro Leu Ser Thr Lys Ser Phe Thr Phe Phe Asn Ser Met Ser Pro Leu
           195                 200                 205
   tcg tcg cag tcc atg ttc tca gca ccc agc tcc atc tcc tcc atg acc     1089
   Ser Ser Gln Ser Met Phe Ser Ala Pro Ser Ser Ile Ser Ser Met Thr
       210                 215                 220
   atg ccg tcc agc atg ggc cca ggc gcc gtg cct ggc atg ccc aac tcg     1137
   Met Pro Ser Ser Met Gly Pro Gly Ala Val Pro Gly Met Pro Asn Ser
   225                 230                 235                 240
   ggc ctc aac aac atc aac aac ctc acc ggc tcc tcg ctc aac tcg gcc     1185
   Gly Leu Asn Asn Ile Asn Asn Leu Thr Gly Ser Ser Leu Asn Ser Ala
                   245                 250                 255
   atg tcg ccg ggc gct tgc ccg tac ggc act ccc gcc tcg ccc tac agc     1233
   Met Ser Pro Gly Ala Cys Pro Tyr Gly Thr Pro Ala Ser Pro Tyr Ser
               260                 265                 270
   gtc tac cgg gac acg tgc aac tcg agc cta gcc agc ctg cgg ctc aag     1281
   Val Tyr Arg Asp Thr Cys Asn Ser Ser Leu Ala Ser Leu Arg Leu Lys
           275                 280                 285
   tcc aaa cag cac tcg tcg ttt ggc tac ggc gcc ctg cag ggc ccg gcc     1329
   Ser Lys Gln His Ser Ser Phe Gly Tyr Gly Ala Leu Gln Gly Pro Ala
   290                 295                 300
   tcg ggc ctc aac gcg tgc cag tac aac agc tga ccgccccgcc gcaccacgcg   1382
   Ser Gly Leu Asn Ala Cys Gln Tyr Asn Ser
   305                 310
   ggccggcggc cggagcgggg aagggcgcgg gcgcggagga cgcacgcggg gccccggctc   1442
   gcaagcccca gctcaccgcg ccgcggacct cacacctgcg cagccccctc ctcccacttc   1502
   ccactccggg ttggttttgt gtttgctttt ccggacccca ctctgccctc caaaaagaca   1562
   aaaaaaaaaa aaaaaaaaaa agcaaaaaga cgtcggagaa aagtgccgcg aaaaaatgga   1622
   tgagttgcaa tttctctcgg gatggcgcgg gtggtgtgtg tgtgttccca cgggccccgg   1682
   aggcccactc cgcggagggc acgcggcgcg gtaggcgagc gccgaggccc agcggccggg   1742
   ggaggacgac ctcgtatccc gcgtccccgc cgcgctggat ccggactgag tggccgggcc   1802
   tgcggactgg atgtgcgggg cctggacttg cctaggattt cccgaccccg tacaaaccaa   1862
   gttgccctct ccgagctagg cccggccgag agcgccttag ctcgagtcgg atccgtgttg   1922
   gggcgggcgt tgggtttggg gggacggtgc ccccagccca ggatcgggca ctcagtggag   1982
   ccgcacacgg ccccggcgcg cctggtagag cctcgctggc cccgcgcccc ggagccctat   2042
   attaaggcca cggagcgaca gcgggcagtg cgggcctggc gggaggtggg ggaggtccat   2102
   ctcagaacac cccagccttg agcttagctg caggcccagg ccctctgctc tgctcccggg   2162
   ctaggaggtg gccctctgtc tgggcgaaca gccccctcct caccgcccgc cgtgcaagag   2222
   tcgagccggc agagcaaggg gcgcggcccc agggccctgc gcccactttg cacacccgct   2282
   ctccggcccg cgcccctgtt tacagcgtcc ctgtgtatgt tggactgact gtaataaatc   2342
   tgtctatatc gactaaaaaa aaaaaaaaaa a                                 2373
<210> 2
   <211> 314
   <212> PRT
   <213> Homo sapiens
<400> 2
   Met Asp Ala Phe Lys Gly Gly Met Ser Leu Glu Arg Leu Pro Glu Gly
   1               5                   10                  15
   Leu Arg Pro Pro Pro Pro Pro Pro His Asp Met Gly Pro Ala Phe His
               20                  25                  30
   Leu Ala Arg Pro Ala Asp Pro Arg Glu Pro Leu Glu Asn Ser Ala Ser
           35                  40                  45
   Glu Ser Ser Asp Thr Glu Leu Pro Glu Lys Glu Arg Gly Gly Glu Pro
       50                  55                  60
   Lys Gly Pro Glu Asp Ser Gly Ala Gly Gly Thr Gly Cys Gly Gly Ala
   65 70 75 80
   Asp Asp Pro Ala Lys Lys Lys Lys Gln Arg Arg Gln Arg Thr His Phe
   85 90 95
   Thr Ser Gln Gln Leu Gln Glu Leu Glu Ala Thr Phe Gln Arg Asn Arg
   100 105 110
   Tyr Pro Asp Met Ser Met Arg Glu Glu Ile Ala Val Trp Thr Asn Leu
   115 120 125
   Thr Glu Pro Arg Val Arg Val Trp Phe Lys Asn Arg Arg Ala Lys Trp
   130 135 140
   Arg Lys Arg Glu Arg Asn Gln Gln Leu Asp Leu Cys Lys Gly Gly Tyr
   145 150 155 160
   Val Pro Gln Phe Ser Gly Leu Val Gln Pro Tyr Glu Asp Val Tyr Ala
   165 170 175
   Ala Gly Tyr Ser Tyr Asn Asn Trp Ala Ala Lys Ser Leu Ala Pro Ala
   180 185 190
   Pro Leu Ser Thr Lys Ser Phe Thr Phe Phe Asn Ser Met Ser Pro Leu
   195 200 205
   Ser Ser Gln Ser Met Phe Ser Ala Pro Ser Ser Ile Ser Ser Met Thr
   210 215 220
   Met Pro Ser Ser Met Gly Pro Gly Ala Val Pro Gly Met Pro Asn Ser
   225 230 235 240
   Gly Leu Asn Asn Ile Asn Asn Leu Thr Gly Ser Ser Leu Asn Ser Ala
   245 250 255
   Met Ser Pro Gly Ala Cys Pro Tyr Gly Thr Pro Ala Ser Pro Tyr Ser
   260 265 270
   Val Tyr Arg Asp Thr Cys Asn Ser Ser Leu Ala Ser Leu Arg Leu Lys
   275 280 285
   Ser Lys Gln His Ser Ser Phe Gly Tyr Gly Ala Leu Gln Gly Pro Ala
   290 295 300
   Ser Gly Leu Asn Ala Cys Gln Tyr Asn Ser
   305 310
<210> 3
   <211> 401
   <212> DNA
   <213> Homo sapiens
<220>
   <221> misc_feature
   <222> (201)..(201)
   <223> SNP1 = A/G
<400> 3
   gctctcctgc ctgccaccct tgggctgctg cccttgggag tgctgggaga tgggctcggc     60
   agtgcagagg cagcagcagg aggaagaaga gaactttccc ccactttgga gctagaaggt    120
   gggtcttgag cataatgctc agataaggga agagtcccag gtagtctcgt acatctattt    180
   acccaactcc actcacctcc rtagcaatgc ctcctgccct ccaactcatc aactcaacac    240
   cagctgccag actttgtgcc aggcaccaac cagcagaaac cagtgaggcc agcagccctc    300
   acgaaagctc accatgtggt taagaaacca cccgctgggt gatgagttct gcgtgccaac    360
   agcatgtcac tggagtgtca gtgaccaact gacttcaaag t                         401
<210> 4
   <211> 426
   <212> DNA
   <213> Homo sapiens
<220>
   <221> misc_feature
   <222> (201)..(201)
   <223> SNP3 = C/T
<400> 4
   atggcagtac tgaagcggtg ggcaggcata ctaacgaaaa acagaaacag aacagaaatg     60
   agcaagacca aaatactttt aagttattat ttaaggtctc ctttcaatta ttttctgcaa    120
   ttattgagcc agcagtctat tgttattaat tcacagtcaa cctatttaag cggtaccagc    180
   aatgcaggca aatgcctcag yaatttccta agttttgggt gttattttct tgtcagttaa    240
   atttaaagca acccatttgt gaccacttta taccaagtta atctcatcta aagtgcttca    300
   cagaccctaa cagctacact aaggttttat actaatgaaa gcaatttaca taaatgaaat    360
   ttatgtcact gcatttcatg acaaattttt tattttataa taattatggt acttatatga    420
   taacat                                                                426
<210> 5
   <211> 611
   <212> DNA
   <213> Homo sapiens
<220>
   <221> misc_feature
   <222> (85)..(85)
   <223> SNP4 = A/G
<400> 5
   catgccactc aaatgttaac aggagggtat cattcctatt agaatgagag gcctggtgga 60
   tgtggatctc tgtggagtat gagtraccta taaatgttaa acccttctat ttaaagctca 120
   cagttttaaa cacatagaaa aatggaagag actcacccac aagcttacat aacagaatca 180
   tagtttaaga aactccgaat tggccaggtg tggtgactct cgcctgtaat cacagcactt 240
   tgggaggcca agatgggagg atcgcttgag gccaggaatt caagaccagc ctgggcaaca 300
   tagtgagatc ccatctccac aaaaaaaagt aaaattaaaa aatttgccag gggtgggcat 360
   ggtggctcac atctataatc cctgcctgag aaggcagggg ggatcacttg aggtcaggag 420
   ttcaagacca gcctggccaa catggtgaat ccatgttgct actaaaaata caaaaattag 480
   ctgagcgtgg tggcgcatcc ctgtaatccc agctacttga gaggctgaag caggagaatt 540
   gcttgaaccc aggagacgga ggttgcagtg agctgagatc gcaccactgc attccagcct 600
   gggtgacaga g 611
<210> 6
   <211> 401
   <212> DNA
   <213> Homo sapiens
<220>
   <221> misc_feature
   <222> (201)..(201)
   <223> SNP6 = T/G
<400> 6
   cctttctcac tgtgggtctg tgaccacagg atgcagggtc tgaggccaga gggaggacac 60
   ttctcagggg tttggattgg ggaggctcag tgactcctgc cttcttccaa gctgagatct 120
   ccctccatga aatgagtctt ggctacgggg aggggaacat ggggaagcac agttgcccag 180
   catttgccct cagccccacc kttgccatgt ttcttggcca cctttaccac gagtctcctg 240
   cagtagaaga tccctccttt caaggagata ttcaggtgtt gcagcgtgct agggagtgtc 300
   ccttcacaga ccattctttt tttagctgtc ttaccaaaaa gaacagcctg aatgcagaca 360
   gcatgggggc acgcacacac acacacatcc tgtacatcca t 401
<210> 7
   <211> 787
   <212> DNA
   <213> Homo sapiens
<220>
   <221> misc_feature
   <222> (332)..(332)
   <223> SNP7 = A/G
<400> 7
   ctgctgcctg ctgtctaagg aaagtgtggc acccgctctt cccagccaag gccaaaagcc      60
   tcatcctaag gtgggaggca gagctggggc aagagttaag tgactgaact gaggccactc     120
   tgtgccctcc tcctgtcttt tgcaggagcc actctgcagg aagtaggtgc cattgaaccc     180
   ttctcccaag ctaattctgc taattctgct ctccttcagc aagctttcag agtcgggttc     240
   tgattttgca cccctgctgc gaccattttg tattcctatc ttaaatgctg gcagacaatc     300
   tggctgactt gtgtccgaga agcagttggc crtgctggga ttgtgacccc agtgtgtggt     360
   gtattacccc agtgaggagt agatggaccc ggcctgggac aggaaggagg agagaggcta     420
   caggggtgtg tgatggagga agacgtggtg gtgatggaat gagacaccag ttttgagcat     480
   tttctaattt gaggcatcac ccagaaaacc ttcagatcat aggaagagtg tggtttgggg     540
   acatttggtt aaagcagatt gattcctcct cgttggggaa cgtacatgtt ggggtgtggc     600
   tgttgggaga gttctagtgg ggcagggaga tggagtcctg gtgagaactg ccgctggaga     660
   gcctgggaaa ccaggcttgc tgggagaagg cagttgtgaa tctgaagaat aaaccacaca     720
   caaggacatc agtgtgtctg gaaatgtgat aaatgtagag ctgatatggt acatcatttg     780
   gcttttt                                                               787
<210> 8
   <211> 1129
   <212> DNA
   <213> Homo sapiens
<220>
   <221> misc_feature
   <222> (634)..(634)
   <223> SNP21 = C/T
<400> 8
   gtgattgagt ttgtgtgtgg aagaggtggc ttgggaggct tccaggagga aatgtccaag 60
   gaagcagtgt atgtcccaag tttttttttt cctagtcaat tattgtattt tatctctttc 120
   aaagaaggaa gtatcacatt gcgattgtcc ttaaaaaaaa aaaaaaacat gggaggtctg 180
   ggagatagta aaacacaatt aaaagcatag gtctagataa gtccctactc tgctacttac 240
   taaccatatg atcttgtcag aacaaattac tctaagcctc agtttcctca tctccaacaa 300
   gaaaagtacc tatatgatca ttgtgagtat taaagtgcca gcagatagtt tagttgcagt 360
   ctgtgttgct atattaaatt gtccagtgca gttttctgaa ctgttttcat tacttaagtc 420
   aaagcacaat tctaacaaaa actcaaatga tctgcaacaa ttttcagacg acagccagga 480
   cacaggagct cagagaagtt agatatggca gggacaaagt cttagttcaa gcaacacacc 540
   tgatcctctc cagtaaagca gacagggaat gagcattctt accctccaaa cagaaatgat 600
   gaatctctag gtattagcca gcttgggacg caaytaagtt ccagcaaatt tgaagtccag 660
   atacgtccaa gtatatatgg tcatcttgta cgtatatttt atgtaagaat acaaagtatt 720
   ttaagtatac aattatttga aaggcaaaat aatggtaaga gtggttctga actttattga 780
   actttggtta actatataac cctcctttaa cccctgcaat gttaggaatg tacacatgaa 840
   agattcagtg tcataattga aacatacctt gaaatctaat aaaatattta tgtgataggt 900
   aaatatctct gtcttgtgat tcactctgtc tgatgtgaca catttcttaa gcacaacata 960
   gagttggatc ccagaagcag gaatgaccta taaagtcatt gaacaaaatg aaaaatcatc 1020
   tttagggttt ggaaaattga aatgaaaagc aaaaatagtt acaaagctgc atctcaagca 1080
   tcaggcaaaa gagctactac acacatttca gtctgactaa accagttac 1129
<210> 9
   <211> 1458
   <212> DNA
   <213> Homo sapiens
<220>
   <221> misc_feature
   <222> (1258)..(1258)
   <223> SNP22 = C/T
<400> 9
   attgtactct aaagaggtta aaccaactta cacttttcaa taaatgtttc tacatacctt 60
   tgtcaatttg atgggtgaaa aactacacct cattctggtt tacaattgtt aattttaagt 120
   gaagtggatc atattttcaa atacatattg gtaatctgta ttttttcttt taggaaagtc 180
   tgctgctatc ttttgcccat ttttcttttt tttttctttt gagacggagt cttgctcttg 240
   ttgcccaggc tggaatgcaa tggcacaatc tcggctcact gcaaactccg cctcccaggt 300
   tcaagcgatt ctcctgcctc agcctcccaa gaagctgaga ttacaggtgt ctgtcaccac 360
   atctggctaa tttttgtatt tctagtagag atggggtttc gccatgttgg ccaggctggt 420
   ctcgaactcc tgacctcgtg atccgcctgc ctcggcctcc caaagtgctg ggattacagg 480
   tgtgagccac cgcgcctggc ctttgcccat ttttctatca atttgtttac accttttatt 540
   tttttaaaga attctttata tattagggag atgagttcct tatctgtcat attatatggg 600
   ttatagatat ttttttcaga ttattatctt ttgactctga tcaggatact gttggctttg 660
   tagaaagttt tttttattat tgttataacg catttatcaa atatttcctt cagcacttct 720
   gggtttttta acattctttg aaaagtccaa cccataccaa gaccattaaa aatcattcat 780
   aatttctcct agtatttcta tggtttcagt tcatttcttt tctttctttc ctcctcctcc 840
   ctctttctct ccttttgcat acaaatgttt ggttcacttt gatatggatt gaacttttat 900
   ttatttattt attttgagac agggtctcac tatcttaccc agggtggagt gcagtggcat 960
   gatcgtagct cactgtaact tcaaactcct ggattcaaat attccttctg cctcagcctc 1020
   ccaagtagct aggactgcgt acacacacac catgcctgac taagttttaa aaatgttttg 1080
   taggaatggg gttttgctat gttgcctagg ctcgtctcga tctcctggcc tcaagtgatc 1140
   ctcccgcaca ggcctcacaa agtgctagga ttacatgcat gagccaccac gcctagactg 1200
   cacttttatt tcttgccagt tgttgtctca atacagttaa tagaatgact tatatttyct 1260
   ctaatcattt aaagtatgac ttctattgta tactacatgc tgtatatatt tgtgcctatt 1320
   tctgaatcat ctgttttgtt tcactaacct attctttcag tacattgact cagatccttg 1380
   atgtattcaa tgcgttggca ttctatattg tatacattag atgcctcctt gaaaggttgt 1440
   gtgtgaagca aaaacaat 1458
<210> 10
   <211> 426
   <212> DNA
   <213> Homo sapiens
<220>
   <221> misc_feature
   <222> (226)..(226)
   <223> SNP23 = A/G
<400> 10
   atgttatcat ataagtacca taattattat aaaataaaaa atttgtcatg aaatgcagtg 60
   acataaattt catttatgta aattgctttc attagtataa aaccttagtg tagctgttag 120
   ggtctgtgaa gcactttaga tgagattaac ttggtataaa gtggtcacaa atgggttgct 180
   ttaaatttaa ctgacaagaa aataacaccc aaaacttagg aaattrctga ggcatttgcc 240
   tgcattgctg gtaccgctta aataggttga ctgtgaatta ataacaatag actgctggct 300
   caataattgc agaaaataat tgaaaggaga ccttaaataa taacttaaaa gtattttggt 360
   cttgctcatt tctgttctgt ttctgttttt cgttagtatg cctgcccacc gcttcagtac 420
   tgccat 426
<210> 11
   <211> 201
   <212> DNA
   <213> Homo sapiens
<220>
   <221> misc_feature
   <222> (101)..(101)
   <223> SNP24 = C/T
<400> 11
   aactatgatt ctgttatgta agcttgtggg tgagtctctt ccatttttct atgtgtttaa 60
   aactgtgagc tttaaataga agggtttaac atttataggt yactcatact ccacagagat 120
   ccacatccac caggcctctc attctaatag gaatgatacc ctcctgttaa catttgagtg 180
   gcatgttcag ctggtaaata a 201
<210> 12
   <211> 401
   <212> DNA
   <213> Homo sapiens
<220>
   <221> misc_feature
   <222> (201)..(201)
   <223> SNP25 ≡ C/T
<400> 12
   cactgctgca gagacccttc tttggaaggc tactctgagt tcccctaggc tcagcctaag 60
   gggtgctgct aggactggca ggattctgtc gtgtgggcct ggagctcaat ctgggggagt 120
   ggcctgctgg caatgccagg gcaccttgca cagagcccag ggcaggtggc atcatcagca 180
   ctgtgggctg ctgcctcata yggttaggta ggactcttca cttaaaaggt ggcattttct 240
   ctgtcattcc tagccaaatg actaccaggg ccatcaaggt cctggcacca ctctggctta 300
   gccatgatgg aaccatctga aggccccagc agggtagcac agcacaggtg actctccttg 360
   cttcagactg gccagcccag aggcctggca gtgcccactc t 401
<210> 13
   <211> 201
   <212> DNA
   <213> Homo sapiens
<220>
   <221> misc_feature
   <222> (101)..(101)
   <223> SNP26 = C/T
<400> 13
   ctgctctact gcctcatagc aaccttattc tttcttactg aagcagctat tttttcaact 60
   ttaagaacca agccatgatt cccttctgag aggggcctaa ytctgagaag ggggcggtgg 120
   ttgcacagac gggtctcggt tggccagtga gttcactggc accttctcac tgcttggtcc 180
   aggctctgaa gtctgctggc c 201
<210> 14
   <211> 1380
   <212> DNA
   <213> Homo sapiens
<220>
   <221> mise_feature
   <222> (1275)..(1275)
   <223> SNP27 = A/T
<400> 14
   tctttgcctg gctcagtctc acctctactc ctgcataccc atcccagttc tgcctgtctt 60
   ttaaggctga gtaccaggct ccctctaaca ggaagctttc ccagattgct cccattagcc 120
   ttgatttctc ctttctcatg ctgtcaccct tgaccttttg tatcctactt tgtatcagac 180
   cttaattcct ctacccatag atatttattt tcctagcacc agctggggag ggctgtggac 240
   tgcagggtcg tccctgacac ctggatgcca gtgatcagcc tgcctgagat atgccagctt 300
   tagaatcctg atggagggag ggaggaggag ggcagcaaag agacccagca gttattttat 360
   gcctatctgt gacaggcaca gtgccaaaca ccatatgtaa cttttcacat tttccccact 420
   ttacagatga ggaaacaagc tcagagaagt tctgtggcag tgaatgagtg gtggtgctgt 480
   gagttaaacc caggctctgc ctaactccaa agttcgtgct cttaacctcc atgatcttgt 540
   ctctggccct catttcccca ccacacccgg aggtaggctg ggctgggtgg gctgaggcag 600
   ggtcttccta ggccttgctg gccagagcag gaggctggtg aaagcccagg acttaaggga 660
   cttcagaggg ggacgtggtt ccctctcacc caagggggag ctcttgacct gctggaggat 720
   ctggggcagt gtagcatccc tgctgggggc ttggaagtgc cactaccccc aaggaagcaa 780
   agcaagaggc ttccctgggg aacctgtctg gaggggaggg aggttggaca tgttggtcaa 840
   gaaagagcgt cttcactcag aaatcctcca gcagtacgag gtcagtgaga tgaaaatgag 900
   taattttgtg cttctgaagc aacaacctac ttgacagcaa tatttaatgg tcatatccag 960
   ggtcttttca aaccctgaca ggaaatccca agaggtaggc aggtggggcc aatgcccgct 1020
   tgacagaggg ttactggggt tctgtgaggt gacatcatgc cctgggaaga gcccagctca 1080
   cgggctagga tttgggcttc tggactcccc gaggggatcg tgccctcctc caggcccctc 1140
   caccagcctg ggctcagtgg atatccctgc ctggaggggc tgggtgagtc tctgtgagcc 1200
   tggggggctg ggtgggcttc agttgcccca ctgtgggctc tggactgtgg gagagttgag 1260
   aggccttggt ggagwtaatg ccaagcttgg gagaagccag gggttcacca tgaattgtgt 1320
   gtgcctgtac tcattccggc cttggtgaca ataggaatgc cagcttgggg atggaggaag 1380
<210> 15
   <211> 401
   <212> DNA
   <213> Homo sapiens
<220>
   <221> misc_feature
   <222> (201)..(201)
   <223> SNP28 = A/G
<400> 15
   cccccagaag agcatgctgg ggtgagtacc cctgcttcgc cccctgcccc gcgtcctgtc 60
   cctgctcatc tcttagggta ttgactgggc tctgcctctc cccagaggcc aggtaggtgg 120
   caggaattgg gtctggttcc tctcccaggg cctggcccac aaaagattgg tggggagatt 180
   gtaccacagg acaataggct rggtgatcga gggcccagaa tctaggatag ttcctggggt 240
   ggggtggggg tgtggccagg aagactgtca atcatgactc atattcagga caccctgcag 300
   aggtggggac ggaaagctga gctgggaaaa tcccaaatga cgtctaaagt gacctgttga 360
   gaaattttga caggtaagtc ctaataatgc agaaagcggg g 401
<210> 16
   <211> 401
   <212> DNA
   <213> Homo sapiens
<220>
   <221> misc_feature
   <222> (201)..(201)
   <223> SNP29 = C/G
<400> 16
   cgccgtgcct ggcatgccca actcgggcct caacaacatc aacaacctca ccggctcctc 60
   gctcaactcg gccatgtcgc cgggcgcttg cccgtacggc actcccgcct cgccctacag 120
   cgtctaccgg gacacgtgca actcgagcct agccagcctg cggctcaagt ccaaacagca 180
   ctcgtcgttt ggctacggcg scctgcaggg cccggcctcg ggcctcaacg cgtgccagta 240
   caacagctga ccgccccgcc gcaccacgcg ggccggcggc cggagcgggg aagggcgcgg 300
   gcgcgggcgc ggaggacgca cgcggggccc cggctcgcaa gccccagctc accgcgccgc 360
   ggacctcaca cctgcgcagc cccctcctcc cacttcccac t 401
<210> 17
   <211> 401
   <212> DNA
   <213> Homo sapiens
<220>
   <221> misc_feature
   <222> (201)..(201)
   <223> SNP30 = C/T
<400> 17
   acacgtcctc gtagggctgc actaggccgc tgaactgcgg cacgtagcca cccttgcaca 60
   ggtccagctg ctggttacgc tcgcgcttac gccacttggc tcgccggttc ttgaaccaga 120
   cctgggggag gggacgggag aagggtcagg gccgctgcgg gccgggaggg accccacccc 180
   cttccccacc gcctggagcc ytccgtcggc ccgctgccct ccgaacgtcg tttctctcct 240
   tcgaccgata tttccgctcc tgcattccct ccacaatggt tcctttcctg tccccagaaa 300
   atactaattt ctttttccga tctttatcct ctcccgaaat tatatccgtt ttcttctccg 360
   aatacggatt ctcgtttaac agatattcgt tttctttctt c 401
<210> 18
   <211> 401
   <212> DNA
   <213> Homo sapiens
<220>
   <221> misc_feature
   <222> (201)..(201)
   <223> SNP31 = A/G
<400> 18
   cccacccacc cctaccacca taaacctggg caggattagt gagttcagga ttactgagtg 60
   ctgtgtcttc attccaagtc ccactgggct ggccagcctg cctagggagg ggtgagggct 120
   ggggttatgg taaggagtct gtgggccagc tgggattttg gtcgacttgg gaggtgctcc 180
   caatatctga ttaaatattt rcgcccccag ggaagaaatg attctcttgc taccctggca 240
   ggaaagcctt gcttttactg gaaaccaaac gggtgagaag agggagaatg caggctggtc 300
   ttgggtctga gatggcatat cctaaaaggg actgggaagg acatttggct cccggagcca 360
   ccccccacct cacctgacgg gagaccttcc ggaaagggca g 401
<210> 19
   <211> 401
   <212> DNA
   <213> Homo sapiens
<220>
   <221> misc_feature
   <222> (201)..(201)
   <223> SNP32 = C/T
<400> 19
   agaaaattcc ggagaaacgc gccattttgg attgactttt taaaatctcc ggaagaataa 60
   tgttgcatgt agatatttta aattccagac ctacacttcg tacgttaatt ctttcagaat 120
   cgaaagatac agttaaaatt aattcatcct catagatttg gttcaaaaaa atcaatatac 180
   gattatatat ctctctaggt yttaaaaata aaaccaacca aagaccgcgc ttaaaacttt 240
   tgaaaacaaa agtagagttc agcgttgatt atgttttaac caagcaaaca acctctcaga 300
   aaaatcactt agggatgcaa aagagaaaca gatactaaga aagtggagag aaacattcag 360
   ctgtgtatta agaaatgaac gcagaagagg cgcccacacc g 401
<210> 20
   <211> 401
   <212> DNA
   <213> Homo sapiens
<220>
   <221> mise_feature
   <222> (201)..(201)
   <223> SNP33 = G/T
<400> 20
   cctttctcac tgtgggtctg tgaccacagg atgcagggtc tgaggccaga gggaggacac 60
   ttctcagggg tttggattgg ggaggctcag tgactcctgc cttcttccaa gctgagatct 120
   ccctccatga aatgagtctt ggctacgggg aggggaacat ggggaagcac agttgcccag 180
   catttgccct cagccccacc kttgccatgt ttcttggcca cctttaccac gagtctcctg 240
   cagtagaaga tccctccttt caaggagata ttcaggtgtt gcagcgtgct agggagtgtc 300
   ccttcacaga ccattctttt tttagctgtc ttaccaaaaa gaacagcctg aatgcagaca 360
   gcatgggggc acgcacacac acacacatcc tgtacatcca t 401
<210> 21
   <211> 429
   <212> DNA
   <213> Homo sapiens
<220>
   <221> misc_feature
   <222> (229)..(229)
   <223> SNP35 = A/G
<400> 21
   gctgcggcgg gacgtcgggg tccggggcga agagagccag ggcgcggacc gacgtctgct 60
   gcttttctgc ggcattgctg cccgaacgaa cgaacgaacg aacgaacgaa gcggtttcgt 120
   ttaggaaaaa taccctcttg acgcgaagcc acggctgaag tcccgggcca cgcagagggg 180
   ccagcaattc catgggtggt ggggccctcc atccctggac gcagcgggra gcagcgggcg 240
   gccctctctc ctgcccactt ctcctggggg tgggggagcc ctgtgtcacc cgacgctgcg 300
   aggggctgcc acgggcttct caggttgtgc ccttcgcggg gccgtggccc gtgcccccga 360
   ggctgctacc ctcttgaggt gcccggagcc aaagcagagg gttgatcccg ccggttgggt 420
   gaggtagca 429
<210> 22
   <211> 649
   <212> DNA
   <213> Homo sapiens
<220>
   <221> misc_feature
   <222> (204)..(204)
   <223> SNP36 = C/G
<400> 22
   ctctgcaatc acaggcacaa aataggtgct ctctacattt tttccaacct gagaggccat 60
   tctagaaggg tctcaggcca cggttctgtc cagtattcca tgcagatgct gacaggactg 120
   caattaaaaa aatacttgag atgcccaaat gcccaaatag cttctcattt tgctttgact 180
   accaataatt gcacagtgca atasaataat gctcaaatac attaacatct tacttgatcc 240
   tagggggtcc tctctacttt taaagcctca aacttcctcc ctctcacagg tgaaaagggg 300
   agtacaaata cattccctcc cttgctctgc ggatccattc ctacaggtag tcaagactct 360
   gagcttcccc tctgactttc tggcagtgct tcacctctcc ccacagatga gtgcaggaac 420
   aattctaaca gacttcagac tcttccaaca gagccaatcc ctccccatca cgttggagtg 480
   gacttgctac ccacaccatc aacaggcccc tgagaaactt cacagggcag gggcttcttt 540
   ggtaaaacca accctttcct tccaccagct cagagaagtt gttccagata gatgccagga 600
   ttcttggagg agcatggtga ttctggggtg gagcctctga ccctggcca 649
<210> 23
   <211> 704
   <212> DNA
   <213> Homo sapiens
<220>
   <221> misc_feature
   <222> (504)..(504)
   <223> SNP37 = G/T
<400> 23
   aaaggcaggg gccataatgt ctcaccttca tatttcccgt gccaagctgt ggccttctgc 60
   attcatggca gatgagtgga caaaggctga tggactgatg gagaaacaaa gggatagatg 120
   gagcagctgg gcagctcagc aaatgatgct gcaatgatct gcttccaact cacctcaaat 180
   ccatccttct ctctccaggc agagtgggct tttaagatac acatctggcc aggtctctca 240
   ctgttcaaac ccttcatctg ctcctttttg ccttcaggat aacatcccac cctcctatca 300
   aggactatgg agccctgtgg gatctggttc ccacttgatt ctccaacttc ctcttcccct 360
   atgccctgcc ttctcatctg ttccagtgct attatgaagc cacacgttct tcctttatta 420
   tcaagcatac cacagtttat ctcacctcag aggctttgca cagtatattt tcctagggag 480
   gggtccccag gtggtagaaa aackgttaca gccaactcct ccatgtgtca ctcaagaccc 540
   ttcaacagca ggctgcagat ttcctctcca gcactgtgct ccaactgtgt aatggatttc 600
   tgtgtctgcc tccttgactc aacccaaatg aacaagagcc atctatctct gtatctctgc 660
   aatcacaggc acaaaatagg tgctctctac atttttccca acct 704
<210> 24
   <211> 401
   <212> DNA
   <213> Homo sapiens
<220>
   <221> misc_feature
   <222> (201)..(201)
   <223> SNP38 = A/G
<400> 24
   cactgctata gcctcactcc atgctgtgca tcctggcact ccagtctcac tccagtacca 60
   gagagggaac agaacctggg gacttgagat gctctatgtt tatgcctggc cccaattctc 120
   cctgtactca aggcctcaag gctcctggga gtcagcctgc gtgcgagtgt cagttctgcc 180
   cccttagtat tgtgagcctt rttttcttag ccgttcaatg agggatgcag agttcttgcc 240
   tcgcagggtt gtgagggaca gagatgccta tatgtggagg gcctggccct cagccacagc 300
   tcgtgagacc atcccgagca gcccttggta gatcctaggg tcaggctggg gctggctatg 360
   ggagccccta ctggatcccc agggctttgt ggtctctttt c 401
<210> 25
   <211> 627
   <212> DNA
   <213> Homo sapiens
<220>
   <221> misc_feature
   <222> (586)..(586)
   <223> SNP39 = A/G
<400> 25
   ctgctgcagt gagcaggcac ctttgtgcat gggcacctgc taggtgcggc ggtgagagtg      60
   tatgtgcata gtaggtgttt gatgtctgtt gaatgggagc atgcaaggct cagggtaggg     120
   cctcagatgc ttagacaagc tacatgtggc ctccccgaca ccccagggct cttcttgtcc     180
   tccctcacag tagtcttcct agcactctca gaaggaccag cctctctcag ctcatgggga     240
   cccactgccc aggatgctgc tggtgagtgt tggttcctgg gggatacttg acctttctgt     300
   tgcaaacgct tacacccacg cttgtgcaag tacactcaga tcccctgctc tcggccgaac     360
   ctgggggttg tgtgaggatg caggcagcag tgagtgcaga ggaagccaag gagggactta     420
   tggacctcag ggagcccctg atgggcccat cattggccct gatgactgag gtcgggtcga     480
   tgttgctagc accttggata ggtccagccc taagctgggc tgggtctgag cagagtggag     540
   atgagtgaga agcctttcta ctccacgttt tcccacagca tttccratag agcgtgttga     600
   gggggtccca gaaagtgcag ctctcag                                          627
<210> 26
   <211> 1095
   <212> DNA
   <213> Homo sapiens
<220>
   <221> misc_feature
   <222> (691)..(691)
   <223> SNP40 = A/G
<400> 26
   gggctggtcc aggtgactca agagcaacag cttaggggga agagccagag gtaggcaagc      60
   agataggggt gactcccacg atggtgactc ccattgctct gtccagacct ctctactgag     120
   ccccacccaa aggcccactc ctcacctcct actggatgaa cctcaaacat ggctcatcaa 180
   aatggaactc atttgtctcc cttccagacc agctcctcca ccagcacctc caactctgga 240
   gtggctctgc cctcatgcaa gcccctcctc ctccctctcc tttagcatcc atcccattgg 300
   caagtcctgc ccctcctacc ctggttctct tcatgcccct gacactgctc ccaccaggat 360
   cccagcaaga gtgactgaac cagttccccc ttctagaccc ttcttccaca cagtggccac 420
   ggtgatcttc aaatgcaaat ctgacccaat gtgcctataa ccaaagcccc ctccaggtac 480
   agtcccctgg gtagctcatg ggggctgcag ctccagcccc tctcacctgc cccacccagc 540
   tctcactcct cctcctttca ctctcttctc cagccacgct gaccttccct gcaccgcccc 600
   catcataagg cctctgcaca tgctcttccc ctgccacagt cactcttccc agccccttgg 660
   cctagcccgc agtcttcgtg cttcaggtct tctttcatgc aggacgacgc ctccctgacc 720
   acactactcc gtgcccagcc ccgcatgcca ccctgctcat ccctccatcc ccctcactgc 780
   accacccagt accagagagc ccatcccatc ctcagagaac tttctactgg actgtgagct 840
   ccctgagggc aggcattgga ctctgccctg ctcctcaccg ctaccccatg cctggttgac 900
   agcaggaagc tgtgtggggg ctgaacaagt gaaggagcca ctgacggggc aggaagtgga 960
   ggtgggcaag cactgtgagg gctgaggcct gtccactgtg tgtaggggat gcccctcaat 1020
   gccccgggac attccctccc cggggctggt cactggggga agacatcccc tctggggatg 1080
   caccacgggg ctggt 1095
<210> 27
   <211> 36
   <212> DNA
   <213> artificial sequence
<220>
   <223> primer
<400> 27
   tgtaaaacga cggccagtcg gcttggggct ggattc 36
<210> 28
   <211> 21
   <212> DNA
   <213> artificial sequence
<220>
   <223> primer
<400> 28
   tgcgacctgc ggggacaaga g 21
<210> 29
   <211> 35
   <212> DNA
   <213> artificial sequence
<220>
   <223> primer
<400> 29
   tgtaaaacga cggccagtgg agggcagccg cttag 35
<210> 30
   <211> 26
   <212> DNA
   <213> artificial sequence
<220>
   <223> primer
<400> 30
   cgagaacggg aaaaagaaag ctcctg 26
<210> 31
   <211> 37
   <212> DNA
   <213> artificial sequence
<220>
   <223> primer
<400> 31
   tgtaaaacga cggccagtgc ctactgacga ccagcac 37
<210> 32
   <211> 25
   <212> DNA
   <213> artificial sequence
<220>
   <223> primer
<400> 32
   gtgggagagt gaagttctgc ttgtg 25
<210> 33
   <211> 37
   <212> DNA
   <213> artificial sequence
<220>
   <223> primer
<400> 33
   tgtaaaacga cggccagtgc aggagcggaa atatcgg 37
<210> 34
   <211> 26
   <212> DNA
   <213> artificial sequence
<220>
   <223> primer
<400> 34
   cggtgaggtt gttgatgttg ttgagg 26
<210> 35
   <211> 37
   <212> DNA
   <213> artificial sequence
<220>
   <223> primer
<400> 35
   tgtaaaacga cggccagtct cagcacccag ctccatc 37
<210> 36
   <211> 30
   <212> DNA
   <213> artificial sequence
<220>
   <223> primer
<400> 36
   ttttttttgt ctttttggag ggcagagtgg 30
<210> 37
   <211> 7520
   <212> DNA
   <213> Homo sapiens
<400> 37
   aggctggaga aggaggggca ggcggcccct cgacgaggac accgctggga gctgccggaa 60
   cgggccccgg gctctgcccc cgccccggcg ctggctcgaa ggcgcccgct cggtgcgatc 120
   ctgttcggca aacattcact catcctgggc tgttctcgcc agggctgggg acttcgaggc 180
   ggccgagacg ggagttgatt ctaggcgaaa caagtcattt gaggcctgag gtgtgcacga 240
   gccgcccggg actcgcaggc cagatgcgtt tcttttgtga ggccgaggga gaactcggtg 300
   tgtcaccggg gaaggaggga gaggcgcggc gaggccgcgg ggggcgggga ggcggcggga 360
   aggtggctgc ggagggggag ggcgcgggcg aggcagggag ggagggaggg cggcagtgag 420
   ggcgcggcgg cgcgggcggc ttggggctgg attccgcccg cgctccctcg ctcgctcgct 480
   ccctccccag ccccctccca cccaggccca ccccacccag cacccctggc gcagggactg 540
   ctggaacctg gctgtgcgcg ctgtcgcttt aagacagact ctgccggcgc cgtccggagc 600
   cttagaaacc ggccccggat cgcgagccgg agccggagcc ggagccgggg ccggccgggc 660
   tgctgaggcc cgagcggcag gagcgcagcg cggagcgctg agccaggcgc ccagtcgcga 720
   gaagctgccg ccgcctctgc ccgcccggcg ccgcagcccc gggcggtcca tggggcgggc 780
   acggcgtcgc tgcaggcgcc ggcagccctg gagggcagcc gcttaggcgc tgcgctcttg 840
   tccccgcagg tcgcagccag ggcggcgggg cgcgcccagc cccggcccct ggagcgcccg 900
   ccgcggtccc cacctccatg gacgccttca aggggggcat gagcctggag cggctgccgg 960
   aggggctccg gccgccgccg ccgccacccc atgacatggg gcccgccttc cacctggccc 1020
   ggcccgccga cccccgcgag ccgctcgaga actccgccag cgagtcgtct gacacggagc 1080
   tgccaggtaa gcgccgttcc ccgcgcggcg cgcacagagc ctacccgggc gcggagcacg 1140
   gggagctggg agcagggcct gacgcccgcg ccgcgcaacc aagctccctc tccgccccag 1200
   aagcgtcagg agctttcttt ttcccgttct cgacctgacg gtgaacgcgg aaacttacgc 1260
   acacgcgaag gcacttccac gcggtcccca cgcgccaaga gactggcaga tgcacgagcc 1320
   tacagctgga gggttggact gacagcccct cttgatcagg agtaaataga acgggaaaga 1380
   atttgatttg cgcaactggg cgctaaggag cggctcggac gaagggagag gaggagaccg 1440
   agaaaggaag aggggtgacg ggcgagagaa cgcactcaga gagacagaga gggaggttaa 1500
   caccgtgaca gtaggcgaca gaatgaaaga gaccaaagag aaggggcggg cgggaagggt 1560
   aaacgggggg agaggggtca tcaagtagaa gggagacaca gaggacggtg agggcgagcg 1620
   ggccaacagg cgcggccgga ctcccagcaa ggccagccgg aacgccgatc aagggatccg 1680
   gcccacgtgc gcggccgaag cccggagcag cccggctctg gcccccgccc cggctcctgc 1740
   gcgtctccct ccgcgctgcc tttttttgtc cgagctggag cttttttcca gcagcgaact 1800
   gggcccgcgg gtggccggcg cgggcagtgg ggcagcgaat ccgctgccca gcgcagccgg 1860
   ccgggttccg gaggggctcc gagctccccg gtgtgggcgc ctcttctgcg ttcatttctt 1920
   aatacacagc tgaatgtttc tctccacttt cttagtatct gtttctcttt tgcatcccta 1980
   agtgattttt ctgagaggtt gtttgcttgg ttaaaacata atcaacgctg aactctactt 2040
   ttgttttcaa aagttttaag cgcggtcttt ggttggtttt atttttaaaa cctagagaga 2100
   tatataatcg tatattgatt tttttgaacc aaatctatga ggatgaatta attttaactg 2160
   tatctttcga ttctgaaaga attaacgtac gaagtgtagg tctggaattt aaaatatcta 2220
   catgcaacat tattcttccg gagattttaa aaagtcaatc caaaatggcg cgtttctccg 2280
   gaattttctc aaaatccatt tacagtggtg ttatttgggg aatttaaatt atctaactat 2340
   aatgtgtttc tctgggattt aaaaattatc tctacaaggg ccttattggg aaggagttaa 2400
   aaatctatct acaacggtac attatttttt taaatcccag agaaataaat gcttctgtca 2460
   acctggagtt tatacactta cagctctgta tttagttagg ggtgtgtttg tacggagctt 2520
   tctggcttct taatttacat ttgctctgtg tttcttcctg tctgattttg agaatttgtg 2580
   gccctttcag tgtctttagg catagagctg tgagcttttc tgggcttctg tgttttgttt 2640
   ttaaactctt tttctttatg tattaatgtt tctatgactg cgtgtgtttt gtgtagcttc 2700
   aaagagagac agactaaagc ccgaggtcac ctagacagag tctgtctgtc tgggctgcag 2760
   ttggtggggt tttctaggct ttgggactgg aggcctggaa gcaaggccga gaacccggct 2820
   gtgttgtgcc tgcccagtgg ggctcccata cacccctgag gagaagaaag tggagggact 2880
   ttaggctgga gagatctgat cctagttcag aaaagcagag accccctcag atttagaccc 2940
   cctaagagtt cgcaaaacaa acaaacaaaa agcatacatt tgctgggaac atcagcagca 3000
   gggtcagaat ctgagccgag ctaagggtgc aggccctgcc cagccaggaa cgcaggcccg 3060
   cggctccagc tccgattctt gggctcttct ccgcggcaca ccagactaga ggtgcctact 3120
   gacgaccagc acccggcggg cgctttccag tggggacagt tcccaggaac cctagaggga 3180
   gggagggagg gaagaccgcc agcgctggtg gcgagcggtt cggtggagcc gggacggggt 3240
   gcaacggggc gtaagcccag gtgacccgct ccccgtcctg cctactgcag agaaggagcg 3300
   cggcggggaa cccaaggggc ccgaggacag tggtgcggga ggcacgggct gcggcggcgc 3360
   agacgaccca gccaagaaga agaagcagcg gcggcaacgt acgcacttca caagccagca 3420
   gttgcaagag ctagaggcca cgttccagag gaaccgctac cccgacatga gcatgaggga 3480
   ggagatcgcc gtgtggacca acctcaccga gccgcgcgtg cgggtgagca aggggtgcgc 3540
   gcctaagggc acccgcgcag agggctcccg ggccgcctct gctgcagccc tccaaagctt 3600
   agacccacaa gcagaacttc actctcccac cattcagacc tgctccctcc ctccaccgga 3660
   gatccccacc ttaggccctg gcccactagc gtctgccagg cctccaggcg cctcggacct 3720
   tcaacccctt tgaccctcat cctacctgat cctcggacta aggcaacccc agcccaaaga 3780
   cctgtgcccc caggggactc ccccatgccc tatggggccc aggacccact cagatgttag 3840
   caggataccc tgggccagag actgggcagt ggattccctg ccgctgggga cctcgggagc 3900
   tgttggcctt atcccgaaaa cgaaagggaa aggcaggtcc tgtctggctg cctcggagaa 3960
   gcctgaaagg tgcctgggtt tggggctctc aaaacttcct ggagcagtgg tcccatctca 4020
   taagcagatt ctctaaaagc tcaagatatt ctccaccctc cccagccatc agtaggaccc 4080
   tttgccttct gccctttccg gaaggtctcc cgtcaggtga ggtggggggt ggctccggga 4140
   gccaaatgtc cttcccagtc ccttttagga tatgccatct cagacccaag accagcctgc 4200
   attctccctc ttctcacccg tttggtttcc agtaaaagca aggctttcct gccagggtag 4260
   caagagaatc atttcttccc tgggggcgta aatatttaat cagatattgg gagcacctcc 4320
   caagtcgacc aaaatcccag ctggcccaca gactccttac cataacccca gccctcaccc 4380
   ctccctaggc aggctggcca gcccagtggg acttggaatg aagacacagc actcagtaat 4440
   cctgaactca ctaatcctgc ccaggtttat ggtggtaggg gtgggtgggg gtggaatcag 4500
   gtagtgtcag tgctggggac tagggaaagc tgagaagcag ggatctggca aactagctca 4560
   ggaaactcca ctcagaactg gaggaaaatc cgagtgggca gagggcatgc ggtaggcaga 4620
   taggtaagga tgggggagga ctaggaggtt cttaccaaat ttggggggag aaaaggaagg 4680
   gcaggaagat tagaagaatc tagtaagaat agaaaaaatt taaatatttg tggagaaaca 4740
   tatttggcat atatttaggc ggcaggaaag aaattaaata ggaagcaaaa agatcaggta 4800
   tttgagaaaa agaatcaact gtttaagggg aacagtgcag gtcagatatt tggaaaggat 4860
   tcaaaagcct aatatttgag gacgattcaa aaggatcaaa aatgggggat aaacaagaaa 4920
   acagactgag ggagaagaaa gaggttcaga cagtggggtg gactgaacga aaataaacat 4980
   ttggagccaa aaagaaaaca tcaaatattg ccgataaaga tgataacaaa ctatttgagc 5040
   gatgaaatag atcgaagaat tgggggagtc ggaaagtcct atgtctcagg gaggaaaaat 5100
   accttttgta tttgagggag aataaaaaga ctgaaatatt tagtagatag agcgaacctg 5160
   tttgggggag aaggcagagg tgaactggtt gagagaggaa gaaagaaaac gaatatctgt 5220
   taaacgagaa tccgtattcg gagaagaaaa cggatataat ttcgggagag gataaagatc 5280
   ggaaaaagaa attagtattt tctggggaca ggaaaggaac cattgtggag ggaatgcagg 5340
   agcggaaata tcggtcgaag gagagaaacg acgttcggag ggcagcgggc cgacggaagg 5400
   ctccaggcgg tggggaaggg ggtggggtcc ctcccggccc gcagcggccc tgacccttct 5460
   cccgtcccct cccccaggtc tggttcaaga accggcgagc caagtggcgt aagcgcgagc 5520
   gtaaccagca gctggacctg tgcaagggtg gctacgtgcc gcagttcagc ggcctagtgc 5580
   agccctacga ggacgtgtac gccgccggct actcctacaa caactgggcc gccaagagcc 5640
   tggcgccagc gccgctctcc accaagagct tcaccttctt caactccatg agcccgctgt 5700
   cgtcgcagtc catgttctca gcacccagct ccatctcctc catgaccatg ccgtccagca 5760
   tgggcccagg cgccgtgcct ggcatgccca actcgggcct caacaacatc aacaacctca 5820
   ccggctcctc gctcaactcg gccatgtcgc cgggcgcttg cccgtacggc actcccgcct    5880
   cgccctacag cgtctaccgg gacacgtgca actcgagcct agccagcctg cggctcaagt    5940
   ccaaacagca ctcgtcgttt ggctacggcg gcctgcaggg cccggcctcg ggcctcaacg    6000
   cgtgccagta caacagctga ccgccccgcc gcaccacgcg ggccggcggc cggagcgggg    6060
   aagggcgcgg gcgcgggcgc ggaggacgca cgcggggccc cggctcgcaa gccccagctc    6120
   accgcgccgc ggacctcaca cctgcgcagc cccctcctcc cacttcccac tccgggttgg    6180
   ttttgtgttt gcttttccgg accccactct gccctccaaa aagacaaaaa aaaaaaaaaa    6240
   aaaaaaaaag caaaaagacg tcggagaaaa gtgccgcgaa aaaatggatg agttgcaatt    6300
   tctctcggga tggcgcgggt ggtgtgtgtg ttcccacggg ccccggaggc ccactccgcg    6360
   gagggcacgc ggcgcggtag gcgagcgccg aggcccagcg gccgggggag gacgacctcg    6420
   tatcccgcgt ccccgccgcg ctggatccgg actgagtggc cgggcctgcg gactggatgt    6480
   gcggggcctg gacttgccta ggatttcccg accccgtaca aaccaagttg ccctctccga    6540
   gctaggcccg gccgagagcg ccttagctcg agtcggatcc gtgttggggc gggcgttggg    6600
   tttgggggga cggtgccccc agcccaggat cgggcactca gtggagccgc acacggcccc    6660
   ggcgcgcctg gtagagcctc gctggccccg cgccccggag ccctatatta aggccacgga    6720
   gcgacagcgg gcagtgcggg cctggcggga ggtgggggag gtccatctca gaacacccca    6780
   gccttgagct tagctgcagg cccaggccct ctgctctgct cccgggctag gaggtggccc    6840
   tctgtctggg cgaacagccc cctcctcacc gcccgccgtg caagagtcga gccggcagag    6900
   caaggggcgc ggccccaggg ccctgcgccc actttgcaca cccgctctcc ggcccgcgcc    6960
   cctgtttaca gcgtccctgt gtatgttgga ctgactgtaa taaatctgtc tatatcgact    7020
   tgtccatgta cgtctattaa aaccatagtc cgagcgtgct aagcactgct gagccccgac    7080
   tgcttcattg ggctgaggag ggaggcggat agaaagggaa ctgtgggtag aaagggaacc    7140
   gggccccttg ggtaggagca gagcgccgag gcagcccccg ccgaggtctg cctaccccgc    7200
   ttccccacca ccattcttga cctgctcagc ctggctccca tgagggcacc gtcctgcgtt    7260
   gcgagcctgt ccggtgcgca cccgctaagc tgtgcctctc ggcgttgtcc gcgggacgcc    7320
   ttagcgagaa ccccctccct ccgcggctgc gggactctac gggtttcttc ccaggccggg    7380
   tctgcggtga ccctcgaggc cccagggtgc tatggggccc ttgcctgcca ggagcgttcg    7440
   ggcaggggct gctgagagcc tctttatgcc tggggttgcc ttttcttctg tgggcccagc    7500
   aacagcccct cttctctcct                                                7520

## Description

### FIELD OF THE INVENTION

The present invention relates generally to the fields of genetics and medicine.

### BACKGROUND OF THE INVENTION

Autism is a neuropsychiatric developmental disorder characterized by impairments in reciprocal social interaction and verbal and non-verbal communication, restricted and stereotyped patterns of interests and activities, and the presence of developmental abnormalities by 3 years of age (Bailey et al., 1996). In his pioneer description of infantile autism, Kanner (1943) included the following symptoms: impaired language, lack of eye contact, lack of social interaction, repetitive behavior, and a rigid need for routine. He noted that in most cases the child's behavior was abnormal from early infancy. On this basis, he suggested the presence of an inborn, presumably genetic, defect. One year later, Hans Asperger in Germany described similar patients and termed the condition "autistic psychopathy".

Autism is defined using behavioral criteria because, so far, no specific biological markers are known for diagnosing the disease. The clinical picture of autism varies in severity and is modified by many factors, including education, ability and temperament. Furthermore, the clinical picture changes over the course of the development within an individual. In addition, autism is frequently associated with other disorders such as attention deficit disorder, motor in coordination and psychiatric symptoms such as anxiety and depression. There is some evidence that autism may also encompass epileptic, metabolic and immune disorder. In line with the clinical recognition of the variability, there is now general agreement that there is a spectrum of autistic disorders, which includes individuals at all levels of intelligence and language ability and spanning all degrees of severity.

Part of the autism spectrum, but considered a special subgroup, is Asperger syndrome (AS). AS is distinguished from autistic disorder by the lack of a clinically significant delay in language development in the presence of the impaired social interaction and restricted repetitive behaviors, interests, and activities that characterize the autism spectrum disorders (ASDs).

ASDs are types of pervasive developmental disorders (PPD). PPD, "not otherwise specified" (PPD-NOS) is used to categorize children who do not meet the strict criteria for autism but who come close, either by manifesting atypical autism or by nearly meeting the diagnostic criteria in two or three of the key areas.

To standardize the diagnosis of autism, diagnostic criteria have been defined by the World Health Organisation (International Classification of Diseases, 10^{th} Revision (ICD-10), 1992) and the American Psychiatric Association (Diagnostic and Statistical Manual of Mental Disorders, 4^{th} edition (DSM-IV), 1994). An Autism Diagnostic Interview (ADI) has been developed (Le Couteur et al., 1989; Lord et al., 1994). The ADI is the only diagnostic tool available to diagnose ASD that has been standardized, rigorously tested and is universally recognized. The ADI is a scored, semi-structured interview of parents that is based on ICD-10 and DSM-IV criteria for the diagnosis of autism. It focuses on behavior in three main areas: qualities of reciprocal social interaction; communication and language; and restricted and repetitive, stereotyped interests and behaviors. Using these criteria, autism is no longer considered a rare disorder. Higher rates of 10-12 cases per 10,000 individuals have been reported in more recent studies (Gillberg and Wing, 1999) compared to the previously reported prevalence rate of 4-5 patients per 10,000 individuals based on Kanner's criteria (Folstein and Rosen-Sheidley, 2001). Estimates for the prevalence rate of the full spectrum of autistic disorders are 1.5 to 2.5 times higher. Reports of a four times higher occurrence in males compared to females are consistent. Mental retardation is present in between 25% and 40% of cases with ASD (Baird et al. 2000; Chakrabarti and Fombonne, 2001). Additional medical conditions involving the brain are seen in ca. 10% of the population (Gillberg and Coleman, 2000).

The mechanisms underlying the increase in reported cases of autism are unknown. It is highly debated whether this difference reflects an increase in the prevalence of autism, a gradual change in diagnostic criteria, a recognition of greater variability of disease expression, or an increased awareness of the disorder. In addition, there is a widespread public perception that the apparent increase is due primarily to environmentally factors (Nelson, 1991; Rodier and Hyman, 1998). However, it seems likely that most of the increased prevalence can be explained by a broadening of the diagnostic criteria, in combination with a broader application of these criteria.

Although there are effective treatments for ameliorating the disease, there are no cures available and benefits of treatment tend to be modest. Promising results have been obtained for several programs utilizing various behavioral and developmental strategies. Among the most promising are programs based on applied behavior analysis (ABA). Several medications appeared to improve various symptoms associated with autism, thereby increasing individuals' ability to benefit from educational and behavioral interventions. The most extensively studied agents are the dopamine antagonists. Several studies suggest the usefulness of various selective serotonin reuptake inhibitors.

Three twin studies have been performed to estimate heritability of autism (Folstein and Rutter, 1977; Bailey et al., 1995; Steftenburg et al., 1989). All twins who lived in a geographically defined population were sought out. In the combined data 36 monozygotic (MZ) and 30 dizygotic (DZ) twins were studied. The average MZ concordance rate is 70% compared to a DZ rate of 0%. A heritability of more than 90% was calculated from the MZ to DZ concordance ratio and the sibling recurrence risk that has been estimated to be ca 2%-4% (Jorde et al., 1991 Szatmari et al., 1998). Studies of non-autistic relatives have clearly shown that several characteristics of the ASDs are found more often in the parents of autistic children than the parents of controls including social reticence, communication difficulties, preference for routines and difficulty with change (Folstein and Rutter, 1977). Delayed onset of speech and difficulty with reading are also more common in family members of individuals with autism, as are recurrent depression, anxiety disorders, elevated platelet serotonin and increased head circumference (Folstein and Rosen-Sheidley, 2001).

The incidence of autism falls significantly with decreasing degree of relatedness to an affected individual indicating that a single-gene model is unlikely to account for most cases of autism (Jorde et al., 1990). A reported segregation analysis was most consistent with a polygenic mode of inheritance (Jorde et al., 1991). The most parsimonious genetic model is one in which several genes interact with one another to produce the autism phenotype (Folstein and Rosen-Sheidley, 2001).

Considerable indirect evidence indicates a possible role for autoimmunity in autism. One study found more family members with autoimmune diseases in families with an autistic proband compared with control probands (Comi et al., 1999). A few studies reported that haplotypes at the Major Histocompatibility Complex (MHC) locus present in some children with autism, or their mothers, might predipose their autistic children to autoimmunity (Burger and Warren, 1998). In two studies, autoantibodies to certain brain tissues and proteins, including myelin basic protein, neurofilament proteins and vascular epithelium were found more often in autistic children compared to controls (Singh et al., 1993; Connolly et al., 1999; Weizman et al., 1982).

Although most autism cases are consistent with the proposed mechanism of oligogenicity and epistasis, a minority have been seen in association with chromosomal abnormalities and with disorders that have specific etiologies. Smalley (1997) stated that approximately 15 to 37% of cases of autism have a comorbid medical condition, including 5 to 14% with a known genetic disorder or chromosomal anomaly. Chromosome anomalies involving almost all human chromosomes have been reported. These include autosomal aneuploidies, sex-chromosome anomalies, deletions, duplications, translocations, ring chromosomes, inversions and marker chromosomes (Gillberg, 1998). Most common are abnormalities of the Prader Willi/Angehnan Syndrome region on chromosome 15. Association of autism and a Mendelian condition or genetic syndrome included untreated phenylketonuria, fragile X syndrome, tuberous sclerosis and neurofibromatosis. Recently, Carney et al. (2003) identified mutations in the MECP2 (methyl CpG-binding protein 2) gene in two females with autism who do not have manifestations of Rett syndrome caused in 80% of the cases by mutations in the MECP2 gene.

Different groups are conducting genome scans related to autism or the broader phenotypes of ASDs. This approach appears very promising, because it is both systematic and model free. In addition, it has already been shown to be successful. Thus, positive linkage results have been obtained even by analysing comparatively small study groups. More important, some findings have already been replicated. The most consistent result was obtained for chromosome 7q, but there is also considerable overlap on chromosomes 2q and 16p (Folstein and Rosen-Sheidley, 2001). Considerable progress in identifying chromosomal regions have also been made on chromosome 15 and X. Mutations in two X-linked genes encoding neuroligins NLGN3 and NLGN4 have been identified in siblings with autism spectrum disorders (Jamain et al., 2003). Several lines of evidence support the fact that mutations in neuroligins are involved in autistic disorder. First, the reported mutations cause severe alterations of the predicted protein structure. Second, deletions at Xp22.3 that include NLGN4 have been reported in several autistic children. Third, a mutation in NLGN4 appeared *de novo* in one affected individual's mother.

### SUMMARY OF THE INVENTION

The present invention now discloses the identification of a human autism susceptibility gene, which can be used for the diagnosis, prevention and treatment of autism, autism spectrum disorders, and autism-associated disorders, as well as for the screening of therapeutically active drugs.

The present invention more particularly discloses the identification of a human autism susceptibility gene, which can be used for the diagnosis, prevention and treatment of autism and related disorders, as well as for the screening of therapeutically active drugs. The invention more specifically discloses certain alleles of the pituitary homeobox transcription factor 1 (PITX1) gene related to susceptibility to autism and representing novel targets for therapeutic intervention. The present invention relates to particular mutations in the PITX1 gene and expression products, as well as to diagnostic tools and kits based on these mutations. The invention can be used in the diagnosis of predisposition to, detection, prevention and/or treatment of Asperger syndrome, pervasive developmental disorder, childhood disintegrative disorder, mental retardation, anxiety, depression, attention deficit hyperactivity disorders, speech delay or language impairment, epilepsy, metabolic disorder, immune disorder, bipolar disease and other psychiatric and neurological diseases including schizophrenia.

The invention can be used in the diagnosis of predisposition to or protection from, detection, prevention and/or treatment of autism, an autism spectrum disorder, or an autism-associated disorder, the method comprising detecting in a sample from the subject the presence of an alteration in the PITX1 gene or polypeptide, the presence of said alteration being indicative of the presence or predisposition to autism, an autism spectrum disorder, or an autism-associated disorder. The presence of said alteration can also be indicative for protecting from autism.

A particular object of this invention resides in a method of detecting the presence of or predisposition to autism, an autism spectrum disorder, or an autism-associated disorder in a subject, the method comprising detecting the presence of an alteration in the PITX1 gene locus in a sample from the subject, the presence of said alteration being indicative of the presence of or the predisposition to autism, an autism spectrum disorder, or an autism-associated disorder. An alteration being indicative of the presence of or the predisposition to autism, an autism spectrum disorder, or an autism-associated disorder is an alteration with a preferential transmission to autists. Alternatively, an alteration being indicative of the presence of or the predisposition to autism, an autism spectrum disorder, or an autism-associated disorder is an alteration with a higher frequency in autists compared to non affected individuals.

An additional particular object of this invention resides in a method of detecting the protection from autism, an autism spectrum disorder, or an autism-associated disorder in a subject, the method comprising detecting the presence of an alteration in the PITX1 gene locus in a sample from the subject, the presence of said alteration being indicative of the protection from autism, an autism spectrum disorder, or an autism-associated disorder. An alteration being indicative of the protection from autism, an autism spectrum disorder, or an autism-associated disorder is an alteration with a preferential non-transmission to autists. Alternatively, an alteration being indicative of the protection from autism, an autism spectrum disorder, or an autism-associated disorder is an alteration with a lower frequency in autists compared to non affected individuals.

Another particular object of this invention resides in a method of assessing the response of a subject to a treatment of autism, an autism spectrum disorder, or an autism-associated disorder, the method comprising detecting the presence of an alteration in the PITX1 gene locus in a sample from the subject, the presence of said alteration being indicative of a particular response to said treatment.

A further particular object of this invention resides in a method of assessing the adverse effect in a subject to a treatment of autism, an autism spectrum disorder, or an autism-associated disorder, the method comprising detecting the presence of an alteration in the PTTX1 gene locus in a sample from the subject, the presence of said alteration being indicative of an adverse effect to said treatment.

This invention also relates to a method for preventing autism, an autism spectrum disorder, or an autism-associated disorder in a subject, comprising detecting the presence of an alteration in the PITX1 gene locus in a sample from the subject, the presence of said alteration being indicative of the predisposition to autism, an autism spectrum disorder, or an autism-associated disorder; and, administering a prophylactic treatment against autism, an autism spectrum disorder, or an autism-associated disorder.

In a preferred embodiment, said alteration is one or several SNP(s) or a haplotype of SNPs associated with autism. Preferably, said SNP(s) are selected from those disclosed in Tables 1a and 1b, more preferably those disclosed in Tables 3-8. More preferably, said SNP associated with autism can be selected from the group consisting of SNP6 and SNP33. More preferably, said haplotype associated with autism comprises or consists of several SNPs selected from SNPs disclosed in Tables 1a and 1b. Preferably, said SNPs are selected from the group consisting of those disclosed in Tables 3-8. In a preferred embodiment, said haplotype associated with autism comprises or consists of several SNPs selected from the group consisting of SNP6, SNP33, SNP25, SNP27, SNP29, SNP31 and SNP33. In a particular embodiment, said haplotype associated with autism comprises or consists of several SNPs selected from the group consisting of SNP1, SNP3, SNP4, SNP6, SNP7, SNP21 and SNP22. Still more preferably, said haplotype is selected from the haplotypes disclosed in Tables 4, 6, 7 and 8.Optionally, the haplotypes disclosed in the present invention can comprise one or several additonal SNPs. More preferably, said SNP associated with autism can be SNP6 or SNP33. In a most preferred embodiment, said haplotype consists of or comprises SNP24, SNP25 and SNP40, preferably with the alleles 1-2-1, respectively. In another most preferred embodiment, said haplotype consists of or comprises SNP23, SNP25 and SNP33, preferably with the alleles 1-2-1, respectively. In a further most preferred embodiment, said haplotype consists of or comprises SNP25, SNP27, SNP29, SNP31, and SNP33 preferably with the alleles 2-1-1-1-1, respectively.

Preferably, the alteration in the PITX1 gene locus is determined by performing a hydridization assay, a sequencing assay, a microsequencing assay, an oligonucleotide ligation assay, a confirmation based assay, a melting curve analysis, a denaturing high performance liquid chromatography (DHPLC) assay or an allele-specific amplification assay.

A particular aspect of this invention resides in compositions of matter comprising primers, probes, and/or oligonucleotides, which are designed to specifically detect at least one SNP or haplotype associated with autism in the genomic region including the PITX1 gene, or a combination thereof. Preferably, said SNP(s) are selected from those disclosed in Tables 1a and 1b, more preferably those disclosed in Tables 3-8. More preferably, said SNP associated with autism can be selected from the group consisting of SNP6 and SNP33. More preferably, said haplotype associated with autism comprises or consists of several SNPs selected from SNPs disclosed in Tables 1a and 1b. Preferably, said SNPs are selected from the group consisting of those disclosed in Tables 3-8. In a preferred embodiment, said haplotype associated with autism comprises or consists of several SNPs selected from the group consisting of SNP6, SNP33, SNP25, SNP27, SNP29, SNP31 and SNP33. In a particular embodiment, said haplotype associated with autism comprises or consists of several SNPs selected from the group consisting of SNP1, SNP3, SNP4, SNP6, SNP7, SNP21 and SNP22. Still more preferably, said haplotype is selected from the haplotypes disclosed in Tables 4, 6, 7 and 8.Optionally, the haplotypes disclosed in the present invention can comprise one or several additonal SNPs. More preferably, said SNP associated with autism can be SNP6 or SNP33. In a most preferred embodiment, said haplotype consists of or comprises SNP24, SNP25 and SNP40, preferably with the alleles 1-2-1, respectively. In another most preferred embodiment, said haplotype consists of or comprises SNP23, SNP25 and SNP33, preferably with the alleles 1-2-1, respectively. In a further most preferred embodiment, said haplotype consists of or comprises SNP25, SNP27, SNP29, SNP31 and SNP33, preferably with the alleles 2-1-1-1-1, respectively.

The invention also resides in methods of treating autism and/or associated disorders in a subject through a modulation of PITX1 expression or activity. Such treatments use, for instance, PITX1 polypeptides, PITX1 DNA sequences (including antisense sequences and RNAi directed at the PITX1 gene locus), anti- PITX1 antibodies or drugs that modulate PITX1 expression or activity.

The invention also relates to methods of treating individuals who carry deleterious alleles of the PITX1 gene, including pre-symptomatic treatment or combined therapy, such as through gene therapy, protein replacement therapy or through the administration of PITX1 protein mimetics and/or inhibitors.

A further aspect of this invention resides in the screening of drugs for therapy of autism or associated disorder, based on the modulation of or binding to an allele of PITX1 gene associated with autism or associated disorder or gene product thereof

A further aspect of this invention includes antibodies specific of PITX1 polypeptide fragments and derivatives of such antibodies, hybridomas secreting such antibodies, and diagnostic kits comprising those antibodies. More preferably, said antibodies are specific to a PITX1 polypeptide or a fragment thereof comprising an alteration, said alteration modifying the activity of PITX1.

The invention also concerns a PITX1 gene or a fragment thereof comprising an alteration. The invention further concerns a PITX1 polypeptide or a fragment thereof comprising an alteration. Preferably, said alteration modifies the activity of PITX1. In a particular embodiment, said alteration is selected from the mutation disclosed in Table 11.

### LEGEND TO THE FIGURES

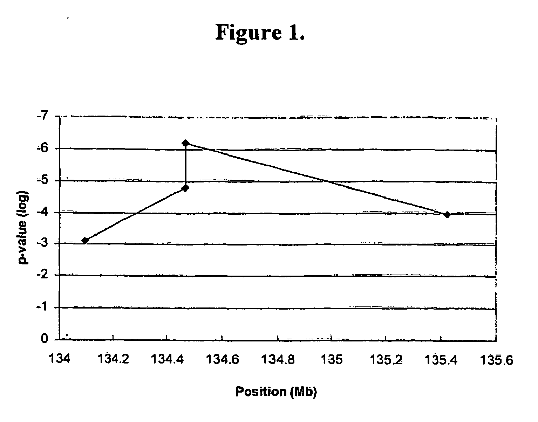

Figure 1 : High density mapping using Genomic Hybrid Identity Profiling (GenomeHIP)
   A total of 2263 BAC clones with an average spacing of 1.2 Mega base pairs between clones representing the whole human genome were tested for linkage using GenomeHIP. Each point corresponds to a clone. Significant evidence for linkage was calculated for clone BACA4ZA08 (p-value 6.4x10⁻⁷). The whole linkage region encompasses a region from 134095595 base pairs to 135593528 base pairs on human chromosome 5. The p-value 2x10⁻⁵ corresponding to the significance level for significant linkage was used as a significance level for whole genome screens as proposed by Lander and Kruglyak (1995).

### DETAILED DESCRIPTION OF THE INVENTION

The present invention discloses the identification of PITX1 as a human autism susceptibility gene. Various nucleic acid samples from 114 families with autism were submitted to a particular GenomeHIP process. This process led to the identification of particular identical-by-descent fragments in said populations that are altered in autistic subjects. By screening of the IBD fragments, we identified the pituitary homeobox transcription factor 1 gene on chromosome 5q31.1 (PITX1) as a candidate for autism and related phenotypes. This gene is indeed present in the critical interval and expresses a functional phenotype consistent with a genetic regulation of autism. SNPs of the PITX1 gene were also identified, as being correlated to autism in human subjects. SNP6 and SNP33, located in the PITX1 gene locus was found to be associated with autism. Haplotypes disclosed in Tables 4, and 6-8 comprising several SNPs selected from the group consisting of SNP1, SNP3, SNP4, SNP6, SNP7, SNP21, SNP22, SNP23, SNP24, SNP25, SNP27, SNP29, SNP31, SNP33, SNP36, SNP39 and SNP40 have also been identified as associated with autism.

The present invention thus proposes to use PITX1 gene and corresponding expression products for the diagnosis, prevention and treatment of autism, autism spectrum disorders, and autism-associated disorders, as well as for the screening of therapeutically active drugs.

### DEFINITIONS

Autism and autism spectrum disorders (ASDs): Autism is typically characterized as part of a spectrum of disorders (ASDs) including Asperger syndrome (AS) and other pervasive developmental disorders (PPD). Autism shall be construed as any condition of impaired social interaction and communication with restricted repetitive and stereotyped patterns of behavior, interests and activities present before the age of 3, to the extent that health may be impaired. AS is distinguished from autistic disorder by the lack of a clinically significant delay in language development in the presence of the impaired social interaction and restricted repetitive behaviors, interests, and activities that characterize the autism-spectrum disorders (ASDs). PPD-NOS (PPD, not otherwise specified) is used to categorize children who do not meet the strict criteria for autism but who come close, either by manifesting atypical autism or by nearly meeting the diagnostic criteria in two or three of the key areas.

Autism-associated disorders, diseases or pathologies include, more specifically, any metabolic and immune disorders, epilepsy, anxiety, depression, attention deficit hyperactivity disorder, speech delay or language impairment, motor incoordination, mental retardation, schizophrenia and bipolar disorder.

The invention may be used in various subjects, particularly human, including adults, children and at the prenatal stage.

Within the context of this invention, the PITX1 gene locus designates all PITX1 sequences or products in a cell or organism, including PITX1 coding sequences, PITX1 non-coding sequences (e.g., introns), PITX1 regulatory sequences controlling transcription, translation and/or stability (e.g., promoter, enhancer, terminator, etc.), as well as all corresponding expression products, such as PITX1 RNAs (e.g., mRNAs) and PITX1 polypeptides (e.g., a pre-protein and a mature protein). The PITX1 gene locus also comprise surrounding sequences of the PITX1 gene which include SNPs that are in linkage disequilibrium with SNPs located in the PITX1 gene. For example, the PITX1 locus comprises surrounding sequences comprising SNPs disclosed in Tables 1a and/or 1b.

As used in the present application, the term "PITX1 gene" designates the pituitary homeobox transcription factor 1 gene on human chromosome 5q31.1, as well as variants, analogs and fragments thereof, including alleles thereof (e.g., germline mutations) which are related to susceptibility to autism and autism-associated disorders. The PITX1 gene may also be referred to as paired-like homeodomain transcription factor pituitary homeobox 1, PTX1, backfoot, mouse, homolog of, BFT, pituitary OTX-related factor, POTX.

The term "gene" shall be construed to include any type of coding nucleic acid, including genomic DNA (gDNA), complementary DNA (cDNA), synthetic or semi-synthetic DNA, as well as any form of corresponding RNA. The term gene particularly includes recombinant nucleic acids encoding PITX1, i.e., any non naturally occurring nucleic acid molecule created artificially, e.g., by assembling, cutting, ligating or amplifying sequences. A PITX1 gene is typically double-stranded, although other forms may be contemplated, such as single-stranded. PITX1 genes may be obtained from various sources and according to various techniques known in the art, such as by screening DNA libraries or by amplification from various natural sources. Recombinant nucleic acids may be prepared by conventional techniques, including chemical synthesis, genetic engineering, enzymatic techniques, or a combination thereof. Suitable PITX1 gene sequences may be found on gene banks, such as Unigene Cluster for PITX1 (Hs.84136) and Unigene Representative Sequence NM_002653. A particular example of a PITX1 gene comprises SEQ ID No: 1 or 37.

The term "PITX1 gene" includes any variant, fragment or analog of SEQ ID No 1 or 37 or of any coding sequence as identified above. Such variants include, for instance, naturally-occurring variants due to allelic variations between individuals (e.g., polymorphisms), mutated alleles related to autism, alternative splicing forms, etc. The term variant also includes PITX1 gene sequences from other sources or organisms. Variants are preferably substantially homologous to SEQ ID No 1 or 37, i.e., exhibit a nucleotide sequence identity of at least about 65%, typically at least about 75%, preferably at least about 85%, more preferably at least about 95% with SEQ ID No 1 or 37. Variants and analogs of a PITX1 gene also include nucleic acid sequences, which hybridize to a sequence as defined above (or a complementary strand thereof) under stringent hybridization conditions.

Typical stringent hybridisation conditions include temperatures above 30° C, preferably above 35°C, more preferably in excess of 42°C, and/or salinity of less than about 500 mM, preferably less than 200 mM. Hybridization conditions may be adjusted by the skilled person by modifying the temperature, salinity and/or the concentration of other reagents such as SDS, SSC, etc.

A fragment of a PITX1 gene designates any portion of at least about 8 consecutive nucleotides of a sequence as disclosed above, preferably at least about 15, more preferably at least about 20 nucleotides, further preferably of at least 30 nucleotides. Fragments include all possible nucleotide lengths between 8 and 100 nucleotides, preferably between 15 and 100, more preferably between 20 and 100.

A PITX1 polypeptide designates any protein or polypeptide encoded by a PITX1 gene as disclosed above. The term "polypeptide" refers to any molecule comprising a stretch of amino acids. This term includes molecules of various lengths, such as peptides and proteins. The polypeptide may be modified, such as by glycosylations and/or acetylatibns and/or chemical reaction or coupling, and may contain one or several non-natural or synthetic amino acids. A specific example of a PITX1 polypeptide comprises all or part of SEQ ID No: 2 (NP_002644).

The terms "response to a treatment" refer to treatment efficacy, including but not limited to ability to metabolise a therapeutic compound, to the ability to convert a pro-drug to an active drug, and to the pharmacokinetics (absorption, distribution, elimination) and the pharmacodynamics (receptor-related) of a drug in an individual.

The terms "adverse effects to a treatment" refer to adverse effects of therapy resulting from extensions of the principal pharmacological action of the drug or to idiosyncratic adverse reactions resulting from an interaction of the drug with unique host factors. "Side effects to a treatment" include, but are not limited to, adverse reactions such as dermatologic, hematologic or hepatologic toxicities and further includes gastric and intestinal ulceration, disturbance in platelet function, renal injury, generalized urticaria, bronchoconstriction, hypotension, and shock.

### DIAGNOSIS

The invention now provides diagnosis methods based on a monitoring of the PITX1 gene locus in a subject. Within the context of the present invention, the term 'diagnosis" includes the detection, monitoring, dosing, comparison, etc., at various stages, including early, pre-symptomatic stages, and late stages, in adults, children and pre-birth. Diagnosis typically includes the prognosis, the assessment of a predisposition or risk of development, the characterization of a subject to define most appropriate treatment (pharmacogenetics), etc.

The present invention provides diagnostic methods to determine whether an individual is at risk of developing autism, an autism spectrum disorder, or an autism-associated disorder or suffers from autism, an autism spectrum disorder, or an autism-associated disorder resulting from a mutation or a polymorphism in the PITX1 gene locus. The present invention also provides methods to determine whether an individual is likely to respond positively to a therapeutic agent or whether an individual is at risk of developing an adverse side effect to a therapeutic agent.

A particular object of this invention resides in a method of detecting the presence of or predisposition to autism, an autism spectrum disorder, or an autism-associated disorder in a subject, the method comprising detecting in a sample from the subject the presence of an alteration in the PITX1 gene locus in said sample. The presence of said alteration is indicative of the presence or predisposition to autism, an autism spectrum disorder, or an autism-associated disorder. Optionally, said method comprises a previous step of providing a sample from a subject. Preferably, the presence of an alteration in the PITX1 gene locus in said sample is detected through the genotyping of a sample.

Another particular object of this invention resides in a method of detecting the protection from autism, an autism spectrum disorder, or an autism-associated disorder in a subject, the method comprising detecting the presence of an alteration in the PITX1 gene locus in a sample from the subject, the presence of said alteration being indicative of the protection from autism, an autism spectrum disorder, or an autism-associated disorder.

In a preferred embodiment, said alteration is one or several SNP(s) or a haplotype of SNPs associated with autism. Preferably, said SNP(s) are selected from those disclosed in Tables 1a and 1b, more preferably those disclosed in Tables 3-8. More preferably, said SNP associated with autism can be selected from the group consisting of SNP6 and SNP33. More preferably, said haplotype associated with autism comprises or consists of several SNPs selected from SNPs disclosed in Tables 1a and 1b. Preferably, said SNPs are selected from the group consisting of those disclosed in Tables 3-8. In a preferred embodiment, said haplotype associated with autism comprises or consists of several SNPs selected from the group consisting of SNP6, SNP33, SNP25, SNP27, SNP29, SNP31 and SNP33. In a particular embodiment, said haplotype associated with autism comprises or consists of several SNPs selected from the group consisting of SNP1, SNP3, SNP4, SNP6, SNP7, SNP21 and SNP22. Still more preferably, said haplotype is selected from the haplotypes disclosed in Tables 4, 6, 7 and 8.Optionally, the haplotypes disclosed in the present invention can comprise one or several additonal SNPs. More preferably, said SNP associated with autism can be SNP6 or SNP33. In a most preferred embodiment, said haplotype consists of or comprises SNP24, SNP25 and SNP40, preferably with the alleles 1-2-1, respectively. In another most preferred embodiment, said haplotype consists of or comprises SNP23, SNP25 and SNP33, preferably with the alleles 1-2-1, respectively. In a further most preferred embodiment, said haplotype consists of or comprises SNP25, SNP27, SNP29, SNP31, and SNP33 preferably with the alleles 2-1-1-1-1, respectively.

Another particular object of this invention resides in a method of assessing the response of a subject to a treatment of autism, an autism spectrum disorder, or an autism-associated disorder, the method comprising (i) providing a sample from the subject and (ii) detecting the presence of an alteration in the PITX1 gene locus in said sample.

Another particular object of this invention resides in a method of assessing the response of a subject to a treatment of autism, an autism spectrum disorder, or an autism-associated disorder, the method comprising detecting in a sample from the subject the presence of an alteration in the PITX1 gene locus in said sample. The presence of said alteration is indicative of a particular response to said treatment. Preferably, the presence of an alteration in the PITX1 gene locus in said sample is detected through the genotyping of a sample.

A further particular object of this invention resides in a method of assessing the adverse effects of a subject to a treatment of autism, an autism spectrum disorder, or an autism-associated disorder, the method comprising detecting in a sample from the subject the presence of an alteration in the PITX1 gene locus in said sample. The presence of said alteration is indicative of adverse effects to said treatment. Preferably, the presence of an alteration in the PITX1 gene locus in said sample is detected through the genotyping of a sample.

In a preferred embodiment, said alteration is one or several SNP(s) or a haplotype of SNPs associated with autism. Preferably, said SNP(s) are selected from those disclosed in Tables 1a and 1b, more preferably those disclosed in Tables 3-8. More preferably, said SNP associated with autism can be selected from the group consisting of SNP6 and SNP33. More preferably, said haplotype associated with autism comprises or consists of several SNPs selected from SNPs disclosed in Tables 1a and 1b. Preferably, said SNPs are selected from the group consisting of those disclosed in Tables 3-8. In a preferred embodiment, said haplotype associated with autism comprises or consists of several SNPs selected from the group consisting of SNP6, SNP33, SNP25, SNP27, SNP29, SNP31 and SNP33. In a particular embodiment, said haplotype associated with autism comprises or consists of several SNPs selected from the group consisting of SNP1, SNP3, SNP4, SNP6, SNP7, SNP21 and SNP22. Still more preferably, said haplotype is selected from the haplotypes disclosed in Tables 4, 6, 7 and 8.Optionally, the haplotypes disclosed in the present invention can comprise one or several additonal SNPs. More preferably, said SNP associated with autism can be SNP6 or SNP33. In a most preferred embodiment, said haplotype consists of or comprises SNP24, SNP25 and SNP40, preferably with the alleles 1-2-1, respectively. In another most preferred embodiment, said haplotype consists of or comprises SNP23, SNP25 and SNP33, preferably with the alleles 1-2-1, respectively. In a further most preferred embodiment, said haplotype consists of or comprises SNP25, SNP27, SNP29, SNP31 and SNP33, preferably with the alleles 2-1-1-1-1, respectively.

In an additional embodiment, the invention concerns a method for preventing autism, an autism spectrum disorder, or an autism-associated disorder in a subject, comprising detecting the presence of an alteration in the PITX1 gene locus in a sample from the subject, the presence of said alteration being indicative of the predisposition to autism, an autism spectrum disorder, or an autism-associated disorder; and, administering a prophylactic treatment against autism, an autism spectrum disorder, or an autism-associated disorder. Said prophylactic treatment can be a drug administration. Said prophylactic treatment can also be a behavioral therapy.

Diagnostics, which analyse and predict response to a treatment or drug, or side effects to a treatment or drug, may be used to determine whether an individual should be treated with a particular treatment drug. For example, if the diagnostic indicates a likelihood that an individual will respond positively to treatment with a particular drug or behavioral therapy, the drug may be administered to the individual. Conversely, if the diagnostic indicates that an individual is likely to respond negatively to treatment with a particular drug, an alternative course of treatment may be prescribed. A negative response may be defined as either the absence of an efficacious response or the presence of toxic side effects.

Clinical drug trials represent another application for the PITX1 SNPs. One or more PITX1 SNPs indicative of response to a drug or to side effects to a drug may be identified using the methods described above. Thereafter, potential participants in clinical trials of such an agent may be screened to identify those individuals most likely to respond favorably to the drug and exclude those likely to experience side effects. In that way, the effectiveness of drug treatment may be measured in individuals who respond positively to the drug, without lowering the measurement as a result of the inclusion of individuals who are unlikely to respond positively in the study and without risking undesirable safety problems.

Clinical trials to assess the utility of a behavioural therapy are also an application for the PITX1 SNPs. One or more PITX1 SNPs indicative of response to a behavioural therapy or to side effects to a behavioral therapy may be identified using the methods described above. Thereafter, potential participants in clinical trials of such a therapy may be screened to identify those individuals most likely to respond favorably to the therapy and exclude those likely to experience side effects. In that way, the effectiveness of behavioral treatment may be measured in individuals who respond positively to the therapy, without lowering the measurement as a result of the inclusion of individuals who are unlikely to respond positively in the study and without risking undesirable safety problems.

The alteration may be determined at the level of the PITX1 gDNA, RNA or polypeptide. Optionally, the detection is determined by performing a hydridization assay, a sequencing assay, a microsequencing assay, an oligonucleotide ligation assay, a confirmation based assay, a melting curve analysis, a denaturing high performance liquid chromatography (DHPLC) assay (Jones et al, 2000) or an allele-specific amplification assay. In a particular embodiment, the detection is performed by sequencing all or part of the PITX1 gene or by selective hybridisation or amplification of all or part of the PITX1 gene. More preferably a PITX1 gene specific amplification is carried out before the alteration identification step.

An alteration in the PITX1 gene locus may be any form of mutation(s), deletion(s), rearrangement(s) and/or insertions in the coding and/or non-coding region of the locus, alone or in various combination(s). Mutations more specifically include point mutations. Deletions may encompass any region of one, two or more residues in a coding or non-coding portion of the gene locus, such as from two residues up to the entire gene or locus. Typical deletions affect smaller regions, such as domains (introns) or repeated sequences or fragments of less than about 50 consecutive base pairs, although larger deletions may occur as well. Insertions may encompass the addition of one or several residues in a coding or non-coding portion of the gene locus. Insertions may typically comprise an addition of between 1 and 50 base pairs in the gene locus. Rearrangement includes inversion of sequences. The PITX1 gene locus alteration may result in the creation of stop codons, frameshift mutations, amino acid substitutions, particular RNA splicing or processing, product instability, truncated polypeptide production, etc. The alteration may result in the production of a PITX1 polypeptide with altered function, stability, targeting or structure. The alteration may also cause a reduction in protein expression or, alternatively, an increase in said production.

In a particular embodiment of the method according to the present invention, the alteration in the PITX1 gene locus is selected from a point mutation, a deletion and an insertion in the PITX1 gene or corresponding expression product, more preferably a point mutation and a deletion. The alteration may be determined at the level of the PITX1 gDNA, RNA or polypeptide.

In this regard, the present invention now discloses a SNP in the PITX1 gene and certain haplotypes, which include SNPs selected from the group consisting of SNP1, SNP3, SNP4, SNP6, SNP7, SNP21 and SNP22, that are associated with autism. The SNPs are reported in the following Table la.

**Table 1a**

| Nucleotide position in genomic sequence of chromosome 5 (Build34) | SNP identity | dbSNP reference | Polymorphism | Position in locus and type of amino acid change | Sequence Reference |
|---|---|---|---|---|---|
| 134298380 | SNP1 | rs319589 | A/G | 3' of PITX1 locus | 3 |
| 134331549 | SNP3 | rs737587 | C/T | 3' of PITX1 locus | 4 |
| 134344806 | SNP4 | rs737587 | C/T | 3' of PITX1 locus | 5 |
| 134448086 | SNP6 | rs39882 | G/T | near PITX1 promoter region | 6 |
| 134530030 | SNP7 | rs745558 | A/G | 5' of PITX1 locus | 7 |
| 135347578 | SNP21 | rs28792 | A/G | 5' of PITX1 locus | 8 |
| 135368924 | SNP22 | rs248166 | A/G | 5' of PITX1 locus | 9 |

**Table 1b**

| Nucleotide position in genomic sequence of chromosome 5 (Build34) | SNP identity | SNP reference | Polymorphism | Position in locus and type of amino acid change | Sequence Reference |
|---|---|---|---|---|---|
| 134331549 | SNP23 | C 644488_10/ rs319589 | A=1/G=2 | 5' of PITX locus | 10 |
| 134344806 | SNP24 | C 644467_10/ rs737587 | C=1/T=2 | 5' of PITX locus | 11 |
| 134424172 | SNP25 | C 15800861_10/ rs2249596 | C=1/T=2 | 5' of PITX locus | 12 |
| 134428749 | SNP26 | C 1012452_10/ rs28330 | C=1/T=2 | 5' of PITX locus | 13 |
| 134432016 | SNP27 | C 3199522_10/ rs657223 | A=1/T=2 | 5' of PITX locus | 14 |
| 134437236 | SNP28 | rs31210 | A=1/G=2 | 5' of PITX locus | 15 |
| 134440733 | SNP29 | C 1012466_10/ rs479632 | C=1/G=2 | Mis-sense Mutation | 16 |
| 134441306 | SNP30 | C 1012468_20/ rs474853 | C=1/T=2 | Intron | 17 |
| 134442415 | SNP31 | C 27486310_10/ rs3805663 | A=1/G=2 | Intron | 18 |
| 134444616 | SNP32 | C 2036559_10/ rs7700313 | C=1/T=2 | Intron | 19 |
| 134448086 | SNP33 | C 11256661_10/ rs39882 | G=1/T=2 | 3' of PITX1 locus | 20 |
| 134451092 | SNP35 | C 8883433_10/ rs1700488 | A=1/G=2 | 3' of PITX1 locus | 21 |
| 134455172 | SNP36 | C 1012473_10/ rs254550 | C=1/G=2 | 3' of PITX1 locus | 22 |
| 134455527 | SNP37 | rs39881 | G=1/T=2 | 3' of PITX1 locus | 23 |
| 134456746 | SNP38 | C 3199528_10/ rs254551 | A=1/G=2 | 3' of PITX1 locus | 24 |
| 134471341 | SNP39 | rs1875957 | A=1/G=2 | 3' of PITX1 locus | 25 |
| 134498420 | SNP40 | rs254577 | A=1/G=2 | 3' of PITX1 locus | 26 |

In any method according to the present invention, one or several SNP in the PITX1 gene and certain haplotypes comprising SNP in the PITX1 gene and surrounding regions, more particularly those disclosed in the present invention, can be used in combination with other SNP or haplotype associated with autism, an autism spectrum disorder, or an autism-associated disorder and located in other gene(s).

In another variant, the method comprises detecting the presence of an altered PITX1 RNA expression. Altered RNA expression includes the presence of an altered RNA sequence, the presence of an altered RNA splicing or processing, the presence of an altered quantity of RNA, etc. These may be detected by various techniques known in the art, including by sequencing all or part of the PITX1 RNA or by selective hybridisation or selective amplification of all or part of said RNA, for instance.

In a further variant, the method comprises detecting the presence of an altered PITX1 polypeptide expression. Altered PITX1 polypeptide expression includes the presence of an altered polypeptide sequence, the presence of an altered quantity of PITX1 polypeptide, the presence of an altered tissue distribution, etc. These may be detected by various techniques known in the art, including by sequencing and/or binding to specific ligands (such as antibodies), for instance.

As indicated above, various techniques known in the art may be used to detect or quantify altered PITX1 gene or RNA expression or sequence, including sequencing, hybridisation, amplification and/or binding to specific ligands (such as antibodies). Other suitable methods include allele-specific oligonucleotide (ASO), oligonucleotide ligation, allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, denaturing HLPC, melting curve analysis, heteroduplex analysis, RNase protection, chemical or enzymatic mismatch cleavage, ELISA, radio-immunoassays (RIA) and immuno-enzymatic assays (IEMA).

Some of these approaches (e.g., SSCA and CGGE) are based on a change in electrophoretic mobility of the nucleic acids, as a result of the presence of an altered sequence. According to these techniques, the altered sequence is visualized by a shift in mobility on gels. The fragments may then be sequenced to confirm the alteration.

Some others are based on specific hybridisation between nucleic acids from the subject and a probe specific for wild type or altered PITX1 gene or RNA. The probe may be in suspension or immobilized on a substrate. The probe is typically labeled to facilitate detection of hybrids.

Some of these approaches are particularly suited for assessing a polypeptide sequence or expression level, such as Northern blot, ELISA and RIA. These latter require the use of a ligand specific for the polypeptide, more preferably of a specific antibody.

In a particular, preferred, embodiment, the method comprises detecting the presence of an altered PITX1 gene expression profile in a sample from the subject. As indicated above, this can be accomplished more preferably by sequencing, selective hybridisation and/or selective amplification of nucleic acids present in said sample.

### Sequencing

Sequencing can be carried out using techniques well known in the art, using automatic sequencers. The sequencing may be performed on the complete PITX1 gene or, more preferably, on specific domains thereof, typically those known or suspected to carry deleterious mutations or other alterations.

### Amplification

Amplification is based on the formation of specific hybrids between complementary nucleic acid sequences that serve to initiate nucleic acid reproduction.

Amplification may be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Preferred techniques use allele-specific PCR or PCR-SSCP. Amplification usually requires the use of specific nucleic acid primers, to initiate the reaction.

Nucleic acid primers useful for amplifying sequences from the PITX1 gene or locus are able to specifically hybridize with a portion of the PITX1 gene locus that flank a target region of said locus, said target region being altered in certain subjects having autism, an autism spectrum disorder, or an autism-associated disorder. Examples of such target regions are provided in Tables 1a and 1b.

Primers that can be used to amplify PITX1 target region comprising SNPs as identified in Table 1 may be designed based on the sequence of Seq Id No 1 or on the genomic sequence of PITX1. In a particular embodiment, primers may be designed based on the sequence of SEQ ID Nos 3-26.

Another particular object of this invention resides in a nucleic acid primer useful for amplifying sequences from the PITX1 gene or locus including surrounding regions. Such primers are preferably complementary to, and hybridize specifically to nucleic acid sequences in the PITX1 gene locus. Particular primers are able to specifically hybridise with a portion of the PITX1 gene locus that flank a target region of said locus, said target region being altered in certain subjects having autism, an autism spectrum disorder, or an autism-associated disorder.

The invention also relates to a nucleic acid primer, said primer being complementary to and hybridizing specifically to a portion of a PITX1 coding sequence (e.g., gene or RNA) altered in certain subjects having autism, an autism spectrum disorder, or an autism-associated disorder. In this regard, particular primers of this invention are specific for altered sequences in a PITX1 gene or RNA. By using such primers, the detection of an amplification product indicates the presence of an alteration in the PITX1 gene locus. In contrast, the absence of amplification product indicates that the specific alteration is not present in the sample.

Typical primers of this invention are single-stranded nucleic acid molecules of about 5 to 60 nucleotides in length, more preferably of about 8 to about 25 nucleotides in length. The sequence can be derived directly from the sequence of the PITX1 gene locus. Perfect complementarity is preferred, to ensure high specificity. However, certain mismatch may be tolerated.

The invention also concerns the use of a nucleic acid primer or a pair of nucleic acid primers as described above in a method of detecting the presence of or predisposition to autism, an autism spectrum disorder, or an autism-associated disorder in a subject or in a method of assessing the response of a subject to a treatment of autism, an autism spectrum disorder, or an autism-associated disorder.

### Selective hybridization

Hybridization detection methods are based on the formation of specific hybrids between complementary nucleic acid sequences that serve to detect nucleic acid sequence alteration(s).

A particular detection technique involves the use of a nucleic acid probe specific for wild type or altered PITX1 gene or RNA, followed by the detection of the presence of a hybrid. The probe may be in suspension or immobilized on a substrate or support (as in nucleic acid array or chips technologies). The probe is typically labeled to facilitate detection of hybrids.

In this regard, a particular embodiment of this invention comprises contacting the sample from the subject with a nucleic acid probe specific for an altered PITX1 gene locus, and assessing the formation of an hybrid. In a particular, preferred embodiment, the method comprises contacting simultaneously the sample with a set of probes that are specific, respectively, for wild type PITX1 gene locus and for various altered forms thereof. In this embodiment, it is possible to detect directly the presence of various forms of alterations in the PITX1 gene locus in the sample. Also, various samples from various subjects may be treated in parallel.

Within the context of this invention, a probe refers to a polynucleotide sequence which is complementary to and capable of specific hybridisation with a (target portion of a) PITX1 gene or RNA, and which is suitable for detecting polynucleotide polymorphisms associated with PITX1 alleles which predispose to or are associated with autism, an autism spectrum disorder, or an autism-associated disorder. Probes are preferably perfectly complementary to the PITX1 gene, RNA, or target portion thereof. Probes typically comprise single-stranded nucleic acids of between 8 to 1000 nucleotides in length, for instance of between 10 and 800, more preferably of between 15 and 700, typically of between 20 and 500. It should be understood that longer probes may be used as well. A preferred probe of this invention is a single stranded nucleic acid molecule of between 8 to 500 nucleotides in length, which can specifically hybridise to a region of a PITX1 gene or RNA that carries an alteration.

A specific embodiment of this invention is a nucleic acid probe specific for an altered (e.g., a mutated) PITX1 gene or RNA, i.e., a nucleic acid probe that specifically hybridises to said altered PITX1 gene or RNA and essentially does not hybridise to a PITX1 gene or RNA lacking said alteration. Specificity indicates that hybridisation to the target sequence generates a specific signal which can be distinguished from the signal generated through non-specific hybridisation. Perfectly complementary sequences are preferred to design probes according to this invention. It should be understood, however, that certain a certain degree of mismatch may be tolerated, as long as the specific signal may be distinguished from non-specific hybridisation.

Particular examples of such probes are nucleic acid sequences complementary to a target portion of the genomic region including the PITX1 gene or RNA carrying a point mutation as listed in Table 1 above. More particularly, the probes can comprise a sequence selected from the group consisting of SEQ ID Nos 3-26 or a fragment thereof comprising the SNP or a complementary sequence thereof.

The sequence of the probes can be derived from the sequences of the PITX1 gene and RNA as provided in the present application. Nucleotide substitutions may be performed, as well as chemical modifications of the probe. Such chemical modifications may be accomplished to increase the stability of hybrids (e.g., intercalating groups) or to label the probe. Typical examples of labels include, without limitation, radioactivity, fluorescence, luminescence, enzymatic labeling, etc.

The invention also concerns the use of a nucleic acid probe as described above in a method of detecting the presence of or predisposition to autism, an autism spectrum disorder, or an autism-associated disorder in a subject or in a method of assessing the response of a subject to a treatment of autism, an autism spectrum disorder, or an autism-associated disorder.

### Oligonucleotide ligation

The oligonucleotide ligation assay is a method consists of designing 3 specific primers per SNP with two primers carrying the SNP-base specific 3' end and one common primer that starts 5' with the next base in the target sequence. The two allele specific primers carry a tag of unique sequences that determine each allele. Primers are annealed to the target sequence and a ligation reaction will join the allele specific primer with the common primer if the allele specific 3'-base is present. A short fluorescent dye labelled probe homologous to the tag of unique sequence, is then hybridised to the immobilized product enabling the detection of the corresponding allele. An oligo-ligation kit is commercially available (SNPlex, Applied Biosystems, Foster City).

### Specific Ligand Binding

As indicated above, alteration in the PITX1 gene locus may also be detected by screening for alteration(s) in PITX1 polypeptide sequence or expression levels. In this regard, a specific embodiment of this invention comprises contacting the sample with a ligand specific for a PITX1 polypeptide and determining the formation of a complex.

Different types of ligands may be used, such as specific antibodies. In a specific embodiment, the sample is contacted with an antibody specific for a PITX1 polypeptide and the formation of an immune complex is determined. Various methods for detecting an immune complex can be used, such as ELISA, radioimmunoassays (RIA) and immuno-enzymatic assays (IEMA).

Within the context of this invention, an antibody designates a polyclonal antibody, a monoclonal antibody, as well as fragments or derivatives thereof having substantially the same antigen specificity. Fragments include Fab, Fab'2, CDR regions, etc. Derivatives include single-chain antibodies, humanized antibodies, poly-functional antibodies, etc.

An antibody specific for a PITX1 polypeptide designates an antibody that selectively binds a PITX1 polypeptide, namely, an antibody raised against a PITX1 polypeptide or an epitope-containing fragment thereof. Although non-specific binding towards other antigens may occur, binding to the target PITX1 polypeptide occurs with a higher affinity and can be reliably discriminated from non-specific binding.

In a specific embodiment, the method comprises contacting a sample from the subject with (a support coated with) an antibody specific for an altered form of a PITX1 polypeptide, and determining the presence of an immune complex. In a particular embodiment, the sample may be contacted simultaneously, or in parallel, or sequentially, with various (supports coated with) antibodies specific for different forms of a PITX1 polypeptide, such as a wild type and various altered forms thereof.

The invention also concerns the use of a ligand, preferably an antibody, a fragment or a derivative thereof as described above, in a method of detecting the presence of or predisposition to autism, an autism spectrum disorder, or an autism-associated disorder in a subject or in a method of assessing the response of a subject to a treatment of autism, an autism spectrum disorder, or an autism-associated disorder.

The invention also relates to a diagnostic kit comprising products and reagents for detecting in a sample from a subject the presence of an alteration in the PITX1 gene or polypeptide, in the PITX1 gene or polypeptide expression, and/or in PITX1 activity. Said diagnostic kit according to the present invention comprises any primer, any pair of primers, any nucleic acid probe and/or any ligand, preferably antibody, described in the present invention. Said diagnostic kit according to the present invention can further comprise reagents and/or protocols for performing a hybridization, amplification or antigen-antibody immune reaction.

The diagnosis methods can be performed in vitro, ex vivo or in vivo, preferably in vitro or ex vivo. They use a sample from the subject, to assess the status of the PITX1 gene locus. The sample may be any biological sample derived from a subject, which contains nucleic acids or polypeptides. Examples of such samples include fluids, tissues, cell samples, organs, biopsies, etc. Most preferred samples are blood, plasma, saliva, urine, seminal fluid, etc. Pre-natal diagnosis may also be performed by testing fetal cells or placental cells, for instance. The sample may be collected according to conventional techniques and used directly for diagnosis or stored. The sample may be treated prior to performing the method, in order to render or improve availability of nucleic acids or polypeptides for testing. Treatments include, for instant, lysis (e.g., mechanical, physical, chemical, etc.), centrifugation, etc. Also, the nucleic acids and/or polypeptides may be pre-purified or enriched by conventional techniques, and/or reduced in complexity. Nucleic acids and polypeptides may also be treated with enzymes or other chemical or physical treatments to produce fragments thereof. Considering the high sensitivity of the claimed methods, very few amounts of sample are sufficient to perform the assay.

As indicated, the sample is preferably contacted with reagents such as probes, primers or ligands in order to assess the presence of an altered PITX1 gene locus. Contacting may be performed in any suitable device, such as a plate, tube, well, glass, etc. In specific embodiments, the contacting is performed on a substrate coated with the reagent, such as a nucleic acid array or a specific ligand array. The substrate may be a solid or semi-solid substrate such as any support comprising glass, plastic, nylon, paper, metal, polymers and the like. The substrate may be of various forms and sizes, such as a slide, a membrane, a bead, a column, a gel, etc. The contacting may be made under any condition suitable for a complex to be formed between the reagent and the nucleic acids or polypeptides of the sample.

The finding of an altered PITX1 polypeptide, RNA or DNA in the sample is indicative of the presence of an altered PITX1 gene locus in the subject, which can be correlated to the presence, predisposition or stage of progression of autism, an autism spectrum disorder, or an autism-associated disorder. For example, an individual having a germ line PITX1 mutation has an increased risk of developing autism, an autism spectrum disorder, or an autism-associated disorder. The determination of the presence of an altered PITX1 gene locus in a subject also allows the design of appropriate therapeutic intervention, which is more effective and customized. Also, this determination at the pre-symptomatic level allows a preventive regimen to be applied.

### Linkage Disequilibirum

Once a first SNP has been identified in a genomic region of interest, more particularly in PITX1 gene locus, the practitioner of ordinary skill in the art can easily identify additional SNPs in linkage disequilibrium with this first SNP. Indeed, any SNP in linkage disequilibrium with a first SNP associated with autism or an associated disorder will be associated with this trait. Therefore, once the association has been demonstrated between a given SNP and autism or an associated disorder, the discovery of additional SNPs associated with this trait can be of great interest in order to increase the density of SNPs in this particular region.

Identification of additional SNPs in linkage disequilibrium with a given SNP involves: (a) amplifying a fragment from the genomic region comprising or surrounding a first SNP from a plurality of individuals; (b) identifying of second SNPs in the genomic region harboring or surrounding said first SNP; (c) conducting a linkage disequilibrium analysis between said first SNP and second SNPs; and (d) selecting said second SNPs as being in linkage disequilibrium with said first marker. Subcombinations comprising steps (b) and (c) are also contemplated.

Methods to identify SNPs and to conduct linkage disequilibrium analysis can be carried out by the skilled person without undue experimentation by using well-known methods.

These SNPs in linkage disequilibrium can also be used in the methods according to the present invention, and more particularly in the diagnosic methods according to the present invention.

For example, a linkage locus of Crohn's disease has been mapped to a large region spanning 18cM on chromosome 5q31 (Rioux et al., 2000 and 2001). Using dense maps of microsatellite markers and SNPs across the entire region, strong evidence of linkage disequilibrium (LD) was found. Having found evidence of LD, the authors developed an ultra-high-density SNP map and studied a denser collection of markers selected from this map. Multilocus analyses defined a single common risk haplotype characterised by multiple SNPs that were each independently associated using TDT. These SNPs were unique to the risk haplotype and essentially identical in their information content by virtue of being in nearly complete LD with one another. The equivalent properties of these SNPs make it impossible to identify the causal mutation within this region on the basis of genetic evidence alone.

### Causal Mutation

Mutations in the PITX1 gene which are responsible for autism or an associated disorder may be identified by comparing the sequences of the PITX1 gene from patients presenting autism or an associated disorder and control individuals. Based on the identified association of SNPs of PITX1 and autism or an associated disorder, the identified locus can be scanned for mutations. In a preferred embodiment, functional regions such as exons and splice sites, promoters and other regulatory regions of the PITX1 gene are scanned for mutations. Preferably, patients presenting autism or an associated disorder carry the mutation shown to be associated with autism or an associated disorder and controls individuals do not carry the mutation or allele associated with autism or an associated disorder. It might also be possible that patients presenting autism or an associated disorder carry the mutation shown to be associated with autism or an associated disorder with a higher frequency than controls individuals.

The method used to detect such mutations generally comprises the following steps: amplification of a region of the PITX1 gene comprising a SNP or a group of SNPs associated with autism or an associated disorder from DNA samples of the PITX1 gene from patients presenting autism or an associated disorder and control individuals; sequencing of the amplified region; comparison of DNA sequences of the PITX1 gene from patients presenting autism or an associated disorder and control individuals; determination of mutations specific to patients presenting autism or an associated disorder.

Therefore, identification of a causal mutation in the PITX1 gene can be carried out by the skilled person without undue experimentation by using well-known methods.

For example, the causal mutations have been identified in the following examples by using routine methods.

Hugot et al. (2001) applied a positional cloning strategy to identify gene variants with susceptibly to Crohn's disease in a region of chromosome 16 previously found to be linked to susceptibility to Crohn's disease. To refine the location of the potential susceptibility locus 26 microsatellite markers were genotyped and tested for association to Crohn's disease using the transmission disequilibrium test. A borderline significant association was found between one allele of the microsatellite marker D16S136. Eleven additional SNPs were selected from surrounding regions and several SNPs showed significant association. SNP5-8 from this region were found to be present in a single exon of the NOD2/CARD15 gene and shown to be non-synonymous variants. This prompted the authors to sequence the complete coding sequence of this gene in 50 CD patients. Two additional non-synonymous mutations (SNP12 and SNP13) were found. SNP13 was most significant associated (p=6x10-6) using the pedigree transmission disequilibrium test. In another independent study, the same variant was found also by sequencing the coding region of this gene from 12 affected individuals compared to 4 controls (Ogura et al., 2001). The rare allele of SNP13 corresponded to a 1-bp insertion predicted to truncate the NOD2/CARD15 protein. This allele was also present in normal healthy individuals, albeit with significantly lower frequency as compared to the controls.

Similarly, Lesage et al. (2002) performed a mutational analyses of CARD15 in 453 patients with CD, including 166 sporadic and 287 familial cases, 159 patients with ulcerative colitis (UC), and 103 healthy control subjects by systematic sequencing of the coding region. Of 67 sequence variations identified, 9 had an allele frequency >5% in patients with CD. Six of them were considered to be polymorphisms, and three (SNP12-R702W, SNP8-G908R, and SNP13-1007fs) were confirmed to be independently associated with susceptibility to CD. Also considered as potential disease-causing mutations (DCMs) were 27 rare additional mutations. The three main variants (R702W, G908R, and 1007fs) represented 32%, 18%, and 31%, respectively, of the total CD mutations, whereas the total of the 27 rare mutations represented 19% of DCMs. Altogether, 93% of the mutations were located in the distal third of the gene. No mutations were found to be associated with UC. In contrast, 50% of patients with CD carried at least one DCM, including 17% who had a double mutation.

### DRUG SCREENING

The present invention also provides novel targets and methods for the screening of drug candidates or leads. The methods include binding assays and/or functional assays, and may be performed in vitro, in cell systems, in animals, etc.

A particular object of this invention resides in a method of selecting compounds active on autism, an autism spectrum disorder, or an autism-associated disorder, said method comprising contacting in vitro a test compound with a PITX1 gene or polypeptide according to the present invention and determining the ability of said test compound to bind said PITX1 gene or polypeptide. Binding to said gene or polypeptide provides an indication as to the ability of the compound to modulate the activity of said target, and thus to affect a pathway leading to autism, an autism spectrum disorder, or an autism-associated disorder in a subject. In a preferred embodiment, the method comprises contacting in vitro a test compound with a PITX1 polypeptide or a fragment thereof according to the present invention and determining the ability of said test compound to bind said PITX1 polypeptide or fragment. The fragment preferably comprises a binding site of the PITX1 polypeptide. Preferably, said PITX1 gene or polypeptide or a fragment thereof is an altered or mutated PITX1 gene or polypeptide or a fragment thereof comprising the alteration or mutation.

A particular object of this invention resides in a method of selecting compounds active on autism, autism spectrum disorders, and autism-associated disorders, said method comprising contacting in vitro a test compound with a PITX1 polypeptide according to the present invention or binding site-containing fragment thereof and determining the ability of said test compound to bind said PITX1 polypeptide or fragment thereof. Preferably, said PITX1 polypeptide or a fragment thereof is an altered or mutated PITX1 polypeptide or a fragment thereof comprising the alteration or mutation.

In a further particular embodiment, the method comprises contacting a recombinant host cell expressing a PITX1 polypeptide according to the present invention with a test compound, and determining the ability of said test compound to bind said PITX1 and to modulate the activity of PITX1 polypeptide. Preferably, said PITX1 polypeptide or a fragment thereof is an altered or mutated PITX1 polypeptide or a fragment thereof comprising the alteration or mutation.

The determination of binding may be performed by various techniques, such as by labeling of the test compound, by competition with a labeled reference ligand, etc.

A further object of this invention resides in a method of selecting compounds active on autism, an autism spectrum disorder, or an autism-associated disorder, said method comprising contacting in vitro a test compound with a PITX1 polypeptide according to the present invention and determining the ability of said test compound to modulate the activity of said PITX1 polypeptide. Preferably, said PITX1 polypeptide or a fragment thereof is an altered or mutated PITX1 polypeptide or a fragment thereof comprising the alteration or mutation.

A further object of this invention resides in a method of selecting compounds active on autism, an autism spectrum disorder, or an autism-associated disorder, said method comprising contacting in vitro a test compound with a PITX1 gene according to the present invention and determining the ability of said test compound to modulate the expression of said PITX1 gene. Preferably, said PITX1 gene or a fragment thereof is an altered or mutated PITX1 gene or a fragment thereof comprising the alteration or mutation.

In an other embodiment, this invention relates to a method of screening, selecting or identifying active compounds, particularly compounds active on autism, an autism spectrum disorder, or an autism-associated disorder, the method comprising contacting a test compound with a recombinant host cell comprising a reporter construct, said reporter construct comprising a reporter gene under the control of a PITX1 gene promoter, and selecting the test compounds that modulate (e.g. stimulate or reduce) expression of the reporter gene. Preferably, said PITX1 gene promoter or a fragment thereof is an altered or mutated PITX1 gene promoter or a fragment thereof comprising the alteration or mutation.

In a particular embodiment of the methods of screening, the modulation is an inhibition. In another particular embodiment of the methods of screening, the modulation is an activation.

The above screening assays may be performed in any suitable device, such as plates, tubes, dishes, flasks, etc. Typically, the assay is performed in multi-wells plates. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

### PHARMACEUTICAL COMPOSITION, THERAPY

A further object of this invention is a pharmaceutical composition comprising (i) a PITX1 polypeptide or a fragment thereof, a nucleic acid encoding a PITX1 polypeptide or a fragment thereof, a vector or a recombinant host cell as described above and (ii) a pharmaceutically acceptable carrier or vehicle.

The invention also relates to a method of treating or preventing autism, an autism spectrum disorder, or an autism-associated disorder in a subject, the method comprising administering to said subject a functional (e.g., wild-type) PITX1 polypeptide or a nucleic acid encoding the same.

An other embodiment of this invention resides in a method of treating or preventing autism, an autism spectrum disorder, or an autism-associated disorder in a subject, the method comprising administering to said subject a compound that modulates, preferably that activates or mimics, expression or activity of a PITX1 gene or protein according to the present invention. Said compound can be an agonist or an antagonist of PITX1, an antisense or a PNAi of PITX1, an antibody or a fragment or a derivative thereof specific to a PITX1 polypeptide according to the present invention. In a particular embodiment of the method, the modulation is an inhibition. In another particular embodiment of the method, the modulation is an activation.

The invention also relates, generally, to the use of a functional PITX1 polypeptide, a nucleic acid encoding the same, or a compound that modulates expression or activity of a PITX1 gene or protein according to the present invention, in the manufacture of a pharmaceutical composition for treating or preventing autism, an autism spectrum disorder, or an autism-associated disorder in a subject. Said compound can be an agonist or an antagonist of PITX1, an antisense or a RNAi of PITX1, an antibody or a fragment or a derivative thereof specific to a PITX1 polypeptide according to the present invention. In a particular embodiment of the method, the modulation is an inhibition. In another particular embodiment of the method, the modulation is an activation.

The present invention demonstrates the correlation between autism, autism spectrum disorders, and autism-associated disorders and the PITX1 gene locus. The invention thus provides a novel target of therapeutic intervention. Various approaches can be contemplated to restore or modulate the PITX1 activity or function in a subject, particularly those carrying an altered PITX1 gene locus. Supplying wild-type function to such subjects is expected to suppress phenotypic expression of autism, autism spectrum disorders, and autism-associated disorders in a pathological cell or organism. The supply of such function can be accomplished through gene or protein therapy, or by administering compounds that modulate or mimic PITX1 polypeptide activity (e.g., agonists as identified in the above screening assays).

The wild-type PITX1 gene or a functional part thereof may be introduced into the cells of the subject in need thereof using a vector as described above. The vector may be a viral vector or a plasmid. The gene may also be introduced as naked DNA. The gene may be provided so as to integrate into the genome of the recipient host' cells, or to remain extra-chromosomal. Integration may occur randomly or at precisely defined sites, such as through homologous recombination. In particular, a functional copy of the PITX1 gene may be inserted in replacement of an altered version in a cell, through homologous recombination. Further techniques include gene gun, liposome-mediated transfection, cationic lipid-mediated transfection, etc. Gene therapy may be accomplished by direct gene injection, or by administering ex vivo prepared genetically modified cells expressing a functional PITX1 polypeptide.

Other molecules with PITX1 activity (e.g., peptides, drugs, PITX1 agonists, or organic compounds) may also be used to restore functional PITX1 activity in a subject or to suppress the deleterious phenotype in a cell.

Restoration of functional PITX1 gene function in a cell may be used to prevent the development of autism, an autism spectrum disorder, or an autism-associated disorder or to reduce progression of said diseases. Such a treatment may suppress the autism-associated phenotype of a cell, particularly those cells carrying a deleterious allele.

Further aspects and advantages of the present invention will be disclosed in the following experimental section, which should be regarded as illustrative and not limiting the scope of the present application.

### GENE, VECTORS, RECOMBINANT CELLS AND POLYPEPTIDES

A further aspect of this invention resides in novel products for use in diagnosis, therapy or screening. These products comprise nucleic acid molecules encoding a PITX1 polypeptide or a fragment thereof, vectors comprising the same, recombinant host cells and expressed polypeptides.

More particularly, the invention concerns an altered or mutated PITX1 gene or a fragment thereof comprising said alteration or mutation. The invention also concerns nucleic acid molecules encoding an altered or mutated PITX1 polypeptide or a fragment thereof comprising said alteration or mutation. Said alteration or mutation modifies the PITX1 activity. The modified activity can be increased or decreased. The invention further concerns a vector comprising an altered or mutated PITX1 gene or a fragment thereof comprising said alteration or mutation or a nucleic acid molecule encoding an altered or mutated PITX1 polypeptide or a fragment thereof comprising said alteration or mutation, recombinant host cells and expressed polypeptides.

A further object of this invention is a vector comprising a nucleic acid encoding a PITX1 polypeptide according to the present invention. The vector may be a cloning vector or, more preferably, an expression vector, i.e., a vector comprising regulatory sequences causing expression of a PITX1 polypeptide from said vector in a competent host cell.

These vectors can be used to express a PITX1 polypeptide in vitro, ex vivo or in vivo, to create transgenic or "Knock Out" non-human animals, to amplify the nucleic acids, to express antisense RNAs, etc.

The vectors of this invention typically comprise a PITX1 coding sequence according to the present invention operably linked to regulatory sequences, e.g., a promoter, a polyA, etc. The term "operably linked" indicates that the coding and regulatory sequences are functionally associated so that the regulatory sequences cause expression (e.g., transcription) of the coding sequences. The vectors may further comprise one or several origins of replication and/or selectable markers. The promoter region may be homologous or heterologous with respect to the coding sequence, and may provide for ubiquitous, constitutive, regulated and/or tissue specific expression, in any appropriate host cell, including for in vivo use. Examples of promoters include bacterial promoters (T7, pTAC, Trp promoter, etc.), viral promoters (LTR, TK, CMV-IE, etc.), mammalian gene promoters (albumin, PGK, etc), and the like.

The vector may be a plasmid, a virus, a cosmid, a phage, a BAC, a YAC, etc. Plasmid vectors may be prepared from commercially available vectors such as pBluescript, pUC, pBR, etc. Viral vectors may be produced from baculoviruses, retroviruses, adenoviruses, AAVs, etc., according to recombinant DNA techniques known in the art.

In this regard, a particular object of this invention resides in a recombinant virus encoding a PITX1 polypeptide as defined above. The recombinant virus is preferably replication-defective, even more preferably selected from El- and/or E4-defective adenoviruses, Gag-, pol- and/or env-defective retroviruses and Rep- and/or Cap-defective AAVs. Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

A further object of the present invention resides in a recombinant host cell comprising a recombinant PITX1 gene or a vector as defined above. Suitable host cells include, without limitation, prokaryotic cells (such as bacteria) and eukaryotic cells (such as yeast cells, mammalian cells, insect cells, plant cells, etc.). Specific examples include E. coli, Kluyveromyces or Saccharomyces yeasts, mammalian cell lines (e.g., Vero cells, CHO cells, 3T3 cells, COS cells, etc.) as well as primary or established mammalian cell cultures (e.g., produced from fibroblasts, embryonic cells, epithelial cells, nervous cells, adipocytes, etc.).

The present invention also relates to a method for producing a recombinant host cell expressing a PITX1 polypeptide according to the present invention, said method comprising (i) introducing in vitro or ex vivo into a competent host cell a recombinant nucleic acid or a vector as described above, (ii) culturing in vitro or ex vivo the recombinant host cells obtained and (iii), optionally, selecting the cells which express the PITX1 polypeptide.

Such recombinant host cells can be used for the production of PITX1 polypeptides, as well as for screening of active molecules, as described below. Such cells may also be used as a model system to study autism. These cells can be maintained in suitable culture media, such as DMEM, RPMI, HAM, etc., in any appropriate culture device (plate, flask, dish, tube, pouch, etc.).

### EXAMPLES

### 1. GenomeHIP platform to identify the chromosome 5 susceptibility gene

The GenomeHIP platform was applied to allow rapid identification of an autism susceptibility gene.
Briefly, the technology consists of forming pairs from the DNA of related individuals. Each DNA is marked with a specific label allowing its identification. Hybrids are then formed between the two DNAs. A particular process (WO00/53802) is then applied that selects all fragments identical-by-descent (IBD) from the two DNAs in a multi step procedure. The remaining IBD enriched DNA is then scored against a BAC clone derived DNA microarray that allows the positioning of the IBD fraction on a chromosome.

The application of this process over many different families results in a matrix of IBD fractions for each pair from each family. Statistical analyses then calculate the minimal IBD regions that are shared between all families tested. Significant results (p-values) are evidence for linkage of the positive region with the trait of interest (here autism). The linked interval can be delimited by the two most distant clones showing significant p-values.

In the present study, 114 families from the United States (114 independent sib-pairs) concordant for strict autism (as defined by ADI-R) were submitted to the GenomeHIP process. The resulting IBD enriched DNA fractions were then labeled with Cy5 fluorescent dyes and hybridised against a DNA array consisting of 2263 BAC clones covering the whole human genome with an average spacing of 1.2 Mega base pairs. Non-selected DNA labeled with Cy3 was used to normalize the signal values and compute ratios for each clone. Clustering of the ratio results was then performed to determine the IBD status for each clone and pair.

By applying this procedure, several BAC clones were identified (BACA19ZB03, BACA16ZG06, BACA4ZA08 and BACA11ZP12) spanning approximately 1.5 megabases in the region on chromosome 5 (bases 134 095 595 to 135 593 528), which showed significant evidence for linkage to autism (p < 6.40E-07).

Table 2: Linkage results for chromosome 5 in the PITX1 locus: Indicated is the region corresponding to 4 BAC clones with evidence for linkage. The start and stop positions of the clones correspond to their genomic locations based on NCBI Build34 with respect to the start of the chromosome (p-ter).

**Table 2**

| Human chromosome | Clones | Start | End | Proportion of informative pairs | p-value |
|---|---|---|---|---|---|
| 5 | BACA19ZB03 | 134 095 595 | 134 095 947 | 0.9 | 0.00078 |
| 5 | BACA16ZG06 | 134 467 773 | 134 639 867 | 0.91 | 1.60E-05 |
| 5 | BACA4ZA08 | 134 467 793 | 134 639 841 | 0.93 | 6.40E-07 |
| 5 | BACA11ZF12 | 135 422 346 | 135 593 528 | 0.9 | 0.00011 |

### 2. Identification of an autism susceptibility gene on chromosome 5

By screening the aforementioned 1.5 Megabases in the linked chromosomal region, we identified the pituitary homeobox transcription factor 1 (PITX1) gene as a candidate for autism and related phenotypes. This gene is indeed present in the critical interval, with evidence for linkage delimited by the clones outlined above.

The PITX1 gene encodes a member of the RIEG/PITX homeobox family, an expanding family of bicoid-related vertebrate homeobox genes. Members of this family are involved in organ development, in particular, the brain and facies, and left-right asymmetry. Lamonerie et al. (1996) cloned and characterized a mouse transcription factor gene (called Ptx1 by them) on the basis of its ability to activate pituitary transcription of the proopiomelanocortin gene (POMC). Six hormones are derived from the POMC gene: ACTH, lipotropin, alpha-MSH, beta-MSH, endorphin, and one other. ACTH and beta-lipotropin (beta-LPH) are derived from a large precursor peptide. Each of these hormones is known to include smaller peptides having distinct biologic activities: alpha-melanotropin (alpha-MSH) and corticotropin-like intermediate lobe peptide (CLIP) are formed from ACTH; gamma-LPH and beta-endorphin are peptide components of beta-LPH. Beta-MSH is contained within gamma-LPH.

The PTX1, PTX2, and PTX3 genes define a novel family of transcription factors, the PTX subfamily, within the paired-like class of homeodomain factors. In mice, Ptx1 and Ptx2 gene expression has been detected in the area of the pituitary primordium and is maintained throughout development in the Rathke pouch and adult pituitary. Pellegrini-Bouiller et al. (1999) demonstrated the expression of the PTX1, PTX2, and PTX3 genes in the normal human pituitary and in the different types of human pituitary adenomas.

Tremblay et al. (1998) reported that most pituitary hormone-coding gene promoters are activated by Ptx1 and so Ptx1 appears to be a general regulator of pituitary-specific transcription. In addition, Ptx1 action is synergized by cell-restricted transcription factors to confer promoter-specific expression. Antisense RNA experiments performed in alphaT3-1 cells that express the alphaGSU gene showed that expression of endogenous alphaGSU is highly dependent on Ptx1 whereas many other genes are not affected. The only other gene found to be highly dependent on Ptx1 for expression was the gene for the Lim3/Lhx3 transcription factor. Thus, Ptx1 is upstream of Lim3/Lhx3 in a cascade of regulators that appear to work in a combinatorial code to direct pituitary-, lineage-, and promoter-specific transcription.

Shapiro et al. (2004) described naturally occurring variants in regulatory regions of PITX1 in stickleback fish that result in differing patterns of PITX1 gene expression and functional differences in pelvic anatomy. They emphasize that regulatory regions of homeobox genes such as PITX1 may be found at numerous distantly spaced sites and as far as several 100's ofkilobases from the gene.

Szeto et al. (1999) found that Pitx1-deleted mice exhibited striking abnormalities in morphogenesis and growth of the hindlimb. Mice homozygous for the Pitx1 targeted mutation die immediately or shortly after birth. A small number of Pitxl-null mice show embryonic lethality after E11.5. The hindlimb of Pitx1-nul1 mice is significantly shorter. The size of the pelvis is also markedly reduced. Examination of thyroid-stimulating hormone beta, luteinizing hormone beta, and the common glycoprotein alpha subunit expression suggests that both the number of gonadotropes and thyrotropes, as well as the level of luteinizing hormone beta and thyroid-stimulating hormone beta transcripts and protein within the individual cells, are diminished. Interestingly, the level of TSH beta transcripts is most severely reduced in the rostral tip thyrotrope population, which does not require Pit-1 for TSH beta gene activation. Growth hormone expression in somatotropes appears unchanged, whereas the number and expression levels of POMC gene in the intermediate lobe melanotropes appear normal between E15.5 and P0. There is a consistent increase in the levels of both number of, and ACTH transcripts and peptide levels in the anterior pituitary corticotropes.

Chamberlain and Herman (1990) proposed a novel biochemical model in which a subgroup of autistic individuals may have a hypersecretion of pineal melatonin that produces a cascade of biochemical effects including a corresponding hyposecretion of pituitary proopiomelanocortin (POMC) peptides and a hypersecretion of hypothalamic opioid peptides and serotonin (5-HT). Autism may reflect a dysfunction in the pineal-hypothalamic-pituitary-adrenal axis that modulates POMC and 5-HT systems of the brain. This model is consistent with numerous clinical investigations implicating hypersecretion of brain 5-HT and opioid peptides in autism.

Curin et al. (2003) found that individuals with autism have significantly lower serum concentrations of cortisol (p < 10(-6)), and significantly higher concentrations of ACTH (p = 0.002) than control age- and sex-matched subjects. Also, prolactin concentrations in autistic patients with epilepsy were significantly higher when compared with normal subjects. The observed hormonal changes are consistent with a dysfunction of the hypothalamic-pituitary-adrenal axis in individuals with autism.

### 3. Association study

The same families that have been used for the linkage study were also used to test for association between a specific phenotype (here autism) in question and the genetic marker allele or haplotypes containing a specific marker allele using the transmission disequilibrium test (TDT). The TDT is a powerful association test as it is insensitive to population stratification problems in the tested sample. Briefly, the segregation of alleles from heterozygous parents to their affected offspring is tested. The portion of alleles transmitted to the affected offspring compared to the non-transmitted alleles is compared to the ratio expected under random distribution. A significant excess of allele transmission over the expected value is evidence for an association of the respective allele or haplotype with the studied autism phenotype.

An alternative test is the pedigree disequilibrium test (PDT) that remains more powerful even when there is misclassification of unaffected individuals (Martin et al. 2000). Simulations suggest that there may be advantages to using the PDT even if the data consist of independent families without extended family information.

The results of this analysis show that certain alleles of the PITX1 gene are positively associated with autism and therefore increase the susceptibility to disease. In the tested population, the allele G of SNP6 is correlated with autism as determined by TDT (p-value = 0.028) and PDT (p-value = 0.0044). In contrast, the allele T of SNP6 is significantly under-transmitted to autistic individuals showing that this allele helps protect from the disease.

Examples of the transmission of the alleles to autists are given in Table 3.

**Table 3**

| Type of test | SNP | Allele | Frequency of allele transmitted to autists | Frequency of allele not transmitted to autists (Note 1) | p- value |
|---|---|---|---|---|---|
| TDT | SNP6 | G | 0.75 | 0.63 | 0.028 |
| TDT | SNP6 | T | 0.25 | 0.37 | 0.028 |
| PDT | SNP6 | G | | 0.71 | 0.0044 |
| PDT | SNP6 | T | | 0.29 | 0.0044 |

| | | | | | |
|---|---|---|---|---|---|
| Table 3, Note 1: The frequency in this column for the PDT test is the population frequency. | | | | | |

In addition, haplotypes were constructed for SNP1, SNP3, SNP4, SNP6, SNP7, SNP21 and SNP22 to identify the phase for all SNPs.

The results of this analysis in the tested population showed that certain haplotypes, all characterized by the presence of allele G at SNP6 are strongly associated with autism, while certain haplotypes devoid of allele G are preferentially not transmitted to autists. An example is the haplotype A-G-A for SNP3-SNP6-SNP22, p = 3.68 x 10⁻⁵. Haplotypes that carry allele T instead of allele G at SNP6 show significant evidence to be under-represented in autistic subjects. An example is the haplotype A-T-A for SNP3-SNP6-SNP22, p = 0.000218.

Preferential transmission of certain haplotypes was also demonstrated by calculation of an odds ratio. An odds ratio greater than 1 means that the tested genetic allele is associated with the disease and therefore that the allele increases the susceptibility to disease. There is a negative association if the odds ratio is smaller than 1 showing that the tested genetic allele helps to protect from the disease. For example, the odds ratio for transmission of the haplotype A-G-G for SNP3-SNP6-SNP22 is 4.32, with p = 3.62 x 10⁻⁵.

Examples of haplotypes with preferential transmission and non-transmission of SNP6 to autists are given in Table 4.

**Table 4**

| SNPs used to construct haplotype | Haplotype | Frequency of haplotype transmitted to autists | Frequency of haplotype not transmitted to autists | p- value |
|---|---|---|---|---|
| SNP3-SNP6 | A-G | 0.509 | 0.347 | 0.008032 |
| SNP3-SNP6 | A-T | 0.074 | 0.209 | 0.001418 |
| SNP4-SNP6-SNP7 | C-G-G | 0.339 | 0.1491 | 0.000739 |
| SNP4-SNP6-SNP7 | C-T-A | 0 | 0.0590 | 0.001157 |
| SNP3-SNP6-SNP22 | A-G-A | 0.433 | 0.1824 | 3.68E-05 |
| SNP3-SNP6-SNP22 | A-T-A | 0.050 | 0.2098 | 0.000218 |
| SNP3-SNP6-SNP21 | A-G-G | 0.418 | 0.1793 | 3.62E-05 |
| SNP3-SNP6-SNP21 | A-T-G | 0.068 | 0.1825 | 0.005894 |
| SNP1-SNP6-SNP22 | C-G-A | 0.410 | 0.1998 | 0.000619 |
| SNP1-SNP6-SNP22 | C-T-A | 0.058 | 0.2095 | 0.000548 |

To increase the information content and narrow down the interval of association, the SNP density in the PITX1 gene was increased to approximately one SNP every 2.5 kb. Several additional markers showed positive single point results in the PITX1 gene. The strongest association was observerd for marker SNP33 with allele 1 being transmitted more frequently to autists than expected by chance, while allele 2 was preferentially non-transmitted to autists (p=0.001674). Interestingly, this marker is identical to SNP6 which was already found to be associated in the previous analysis. Examples of alleles transmitted and non-transmitted to autists are shown in Table 5.

**Table 5**

| Marker | Allele | N transmitted | N non transmitted | p-value |
|---|---|---|---|---|
| SNP25 | 1 | 55 | 84 | 0.013903 |
| SNP25 | 2 | 84 | 55 | 0.013903 |
| SNP26 | 1 | 100 | 71 | 0.026576 |
| SNP26 | 2 | 71 | 100 | 0.026576 |
| SNP27 | 1 | 98 | 70 | 0.030754 |
| SNP27 | 2 | 70 | 98 | 0.030754 |
| SNP31 | 1 | 100 | 62 | 0.002831 |
| SNP31 | 2 | 62 | 100 | 0.002831 |
| SNP32 | 1 | 19 | 40 | 0.006258 |
| SNP32 | 2 | 40 | 19 | 0.006258 |
| SNP33 | 1 | 101 | 61 | 0.001674 |
| SNP33 | 2 | 61 | 101 | 0.001674 |
| SNP35 | 1 | 15 | 31 | 0.018321 |
| SNP35 | 2 | 31 | 15 | 0.018321 |

Haplotypes were also constructed to determine the phase and analysed for association in the complete sample set used for linkage analysis as described above. The results of this analysis in the tested population showed that certain haplotypes are strongly associated with autism, of which all are characterized by the presence of allele 2 at marker SNP25, allele 1 at marker SNP27, allele 1 at marker SNP29, allele 1 at marker SNP31 or allele 1 at marker SNP33, respectively. The most significant result was obtained for haplotype 1-2-1 for markers SNP24, SNP25 and SNP40 with a p-value of 2.24 x 10⁻⁰³. While certain haplotypes characterised by allele 1 at marker SNP25, allele 2 at marker SNP27 or allele 2 at marker SNP40, respectively, are preferentially not transmitted to autists.

Examples of haplotypes with preferential transmission and non-transmission to autists from the complete data set are given in Table 6.

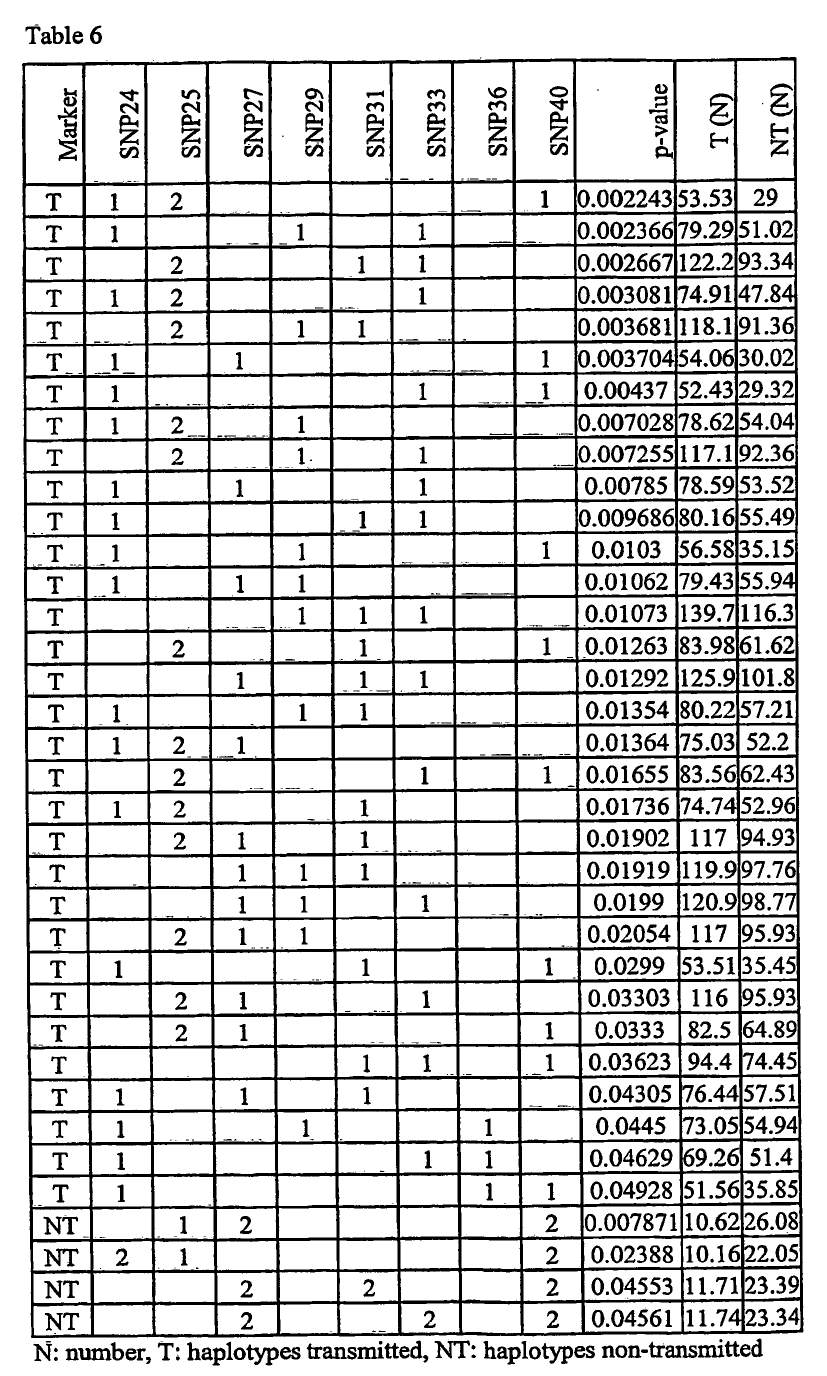

This family set includes samples from different ethnic groups represented within the population of the USA. In order to adjust for any population stratification effects, the haplotype analysis was repeated for each ethnic group. Significant association was found for several haplotypes in the Caucasian population. An example for a haplotype being transmitted more often to autistis is haplotype 1-2-1 for markers SNP23, SNP25 and SNP33 (p=4.44 x 10⁻³), while haplotype 1-2-2 for markers SNP25, SNP29 and SNP31 was preferentially not transmitted to autists (p=0.006).

Examples of haplotypes with preferential transmission and non-transmission to autists from the Caucasian data set are given in Table 7.

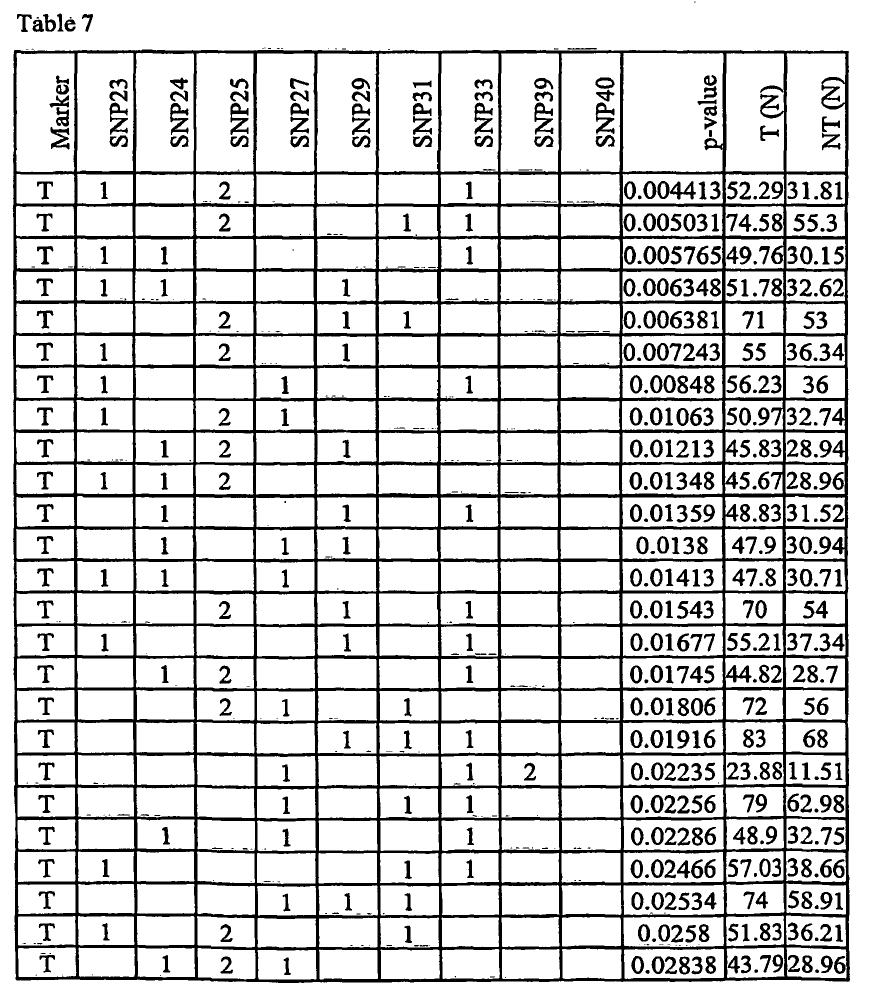

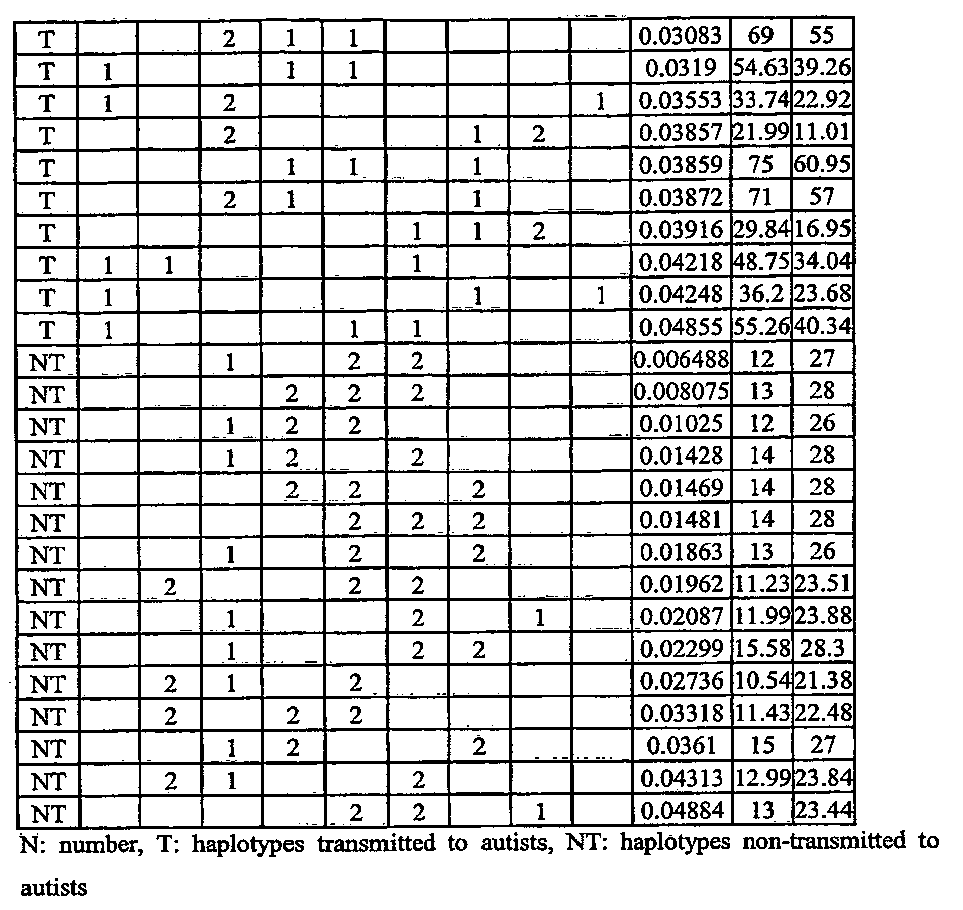

A second independent set of 167 trio families (set 2) was studied for replication of the association that has been observed in the families providing evidence for linkage (set 1) as described above.

As this second family set (set 2) also includes samples from different ethnic groups represented within the population of the USA, like set 1, the haplotype analysis was performed for each ethnic group separately in order to adjust for potential population stratification effects. Significant association was found for several haplotypes in the Caucasian population characterised by the presence of alleles 2, 1, 1, 1, or 1 for markers SNP25, SNP27, SNP29, SNP31 and SNP33. An example for a haplotype being transmitted more often to autistis is haplotype 2-1-2 for markers SNP25, SNP27 and SNP32 (p=5.93 x 10⁻³) while the haplotype 2-2-1 for markers SNP25, SNP26 and SNP27 was more frequently not transmitted to autists (p=0.04).

Examples of haplotypes with preferential transmission and non-transmission to autists from the Caucasian data set in family set 2 are given in Table 8.

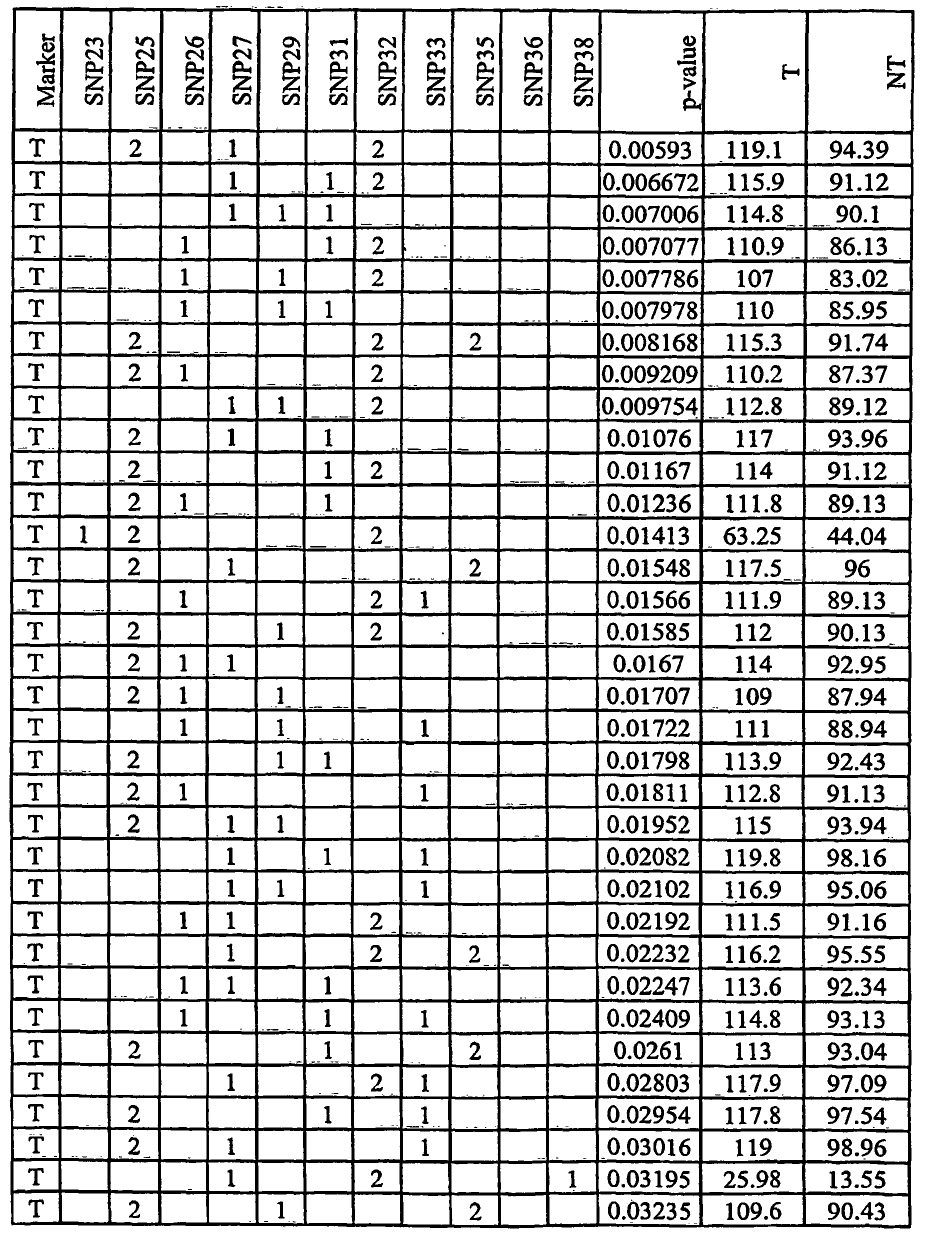

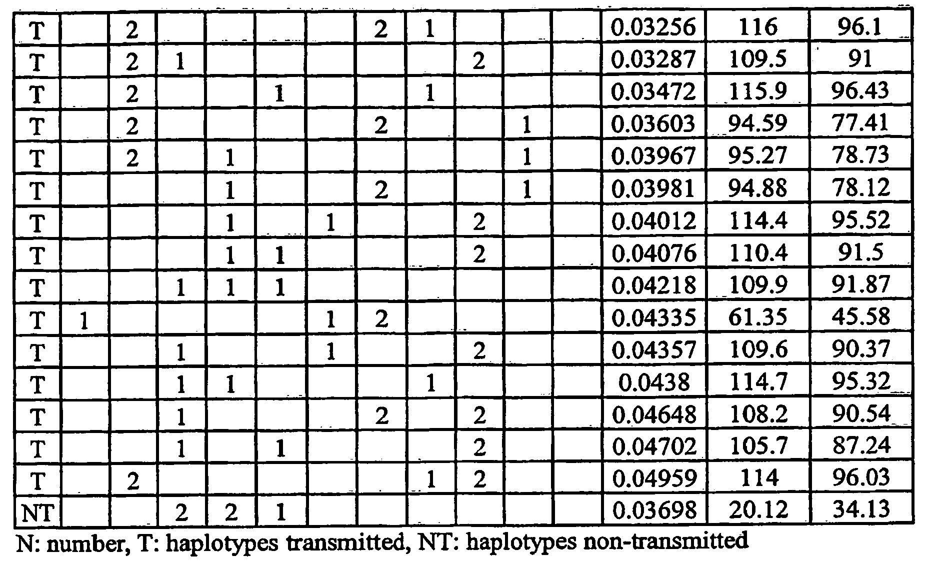

In summary, haplotype analysis in Caucasians showed positive replication of haplotype 2-1-1-1-1 for markers SNP25, SNP27, SNP29, SNP31 and SNP33 in set 2 (p = 3.2 x 10⁻³ in set 1 and p = 6.85 x 10⁻³ in set 2) as shown in Table 9 below.

**Table 9: Haplotype analysis in Caucasians in families from set 1 and set2.**

| Marker | | | | | SET1 | | | SET2 | | |
|---|---|---|---|---|---|---|---|---|---|---|
| SNP25 | SNP27 | SNP29 | SNP31 | SNP33 | p-value | T | NT | p-value | T | NT |
| 2 | 1 | 1 | 1 | 1 | 0.003213 | 69 | 50 | 0.006854 | 113 | 89 |

### 4. Identification of nucleotide changes

96 unrelated affected individuals were included in the mutation screen. Primers were designed to amplify the coding region of the PITX1 gene. The sequences of the primers are provided below in Table 10:

**Table 10**

| | Forward primer | Reverse primer |
|---|---|---|
| Exon 1 | SEQ ID NO: 27 | SEQ ID NO: 28 |
| Exon 2 | SEQ ID NO: 29 | SEQ ID NO: 30 |
| Exon 3 | SEQ ID NO: 31 | SEQ ID NO: 32 |
| Exon 4a | SEQ ID NO: 33 | SEQ ID NO: 34 |
| Exon 4b | SEQ ID NO: 35 | SEQ ID NO: 36 |

The resulting amplification products were directly sequenced in one direction using dye-terminator sequencing chemistry to identify rare nucleotide changes (mutations) and polymorphisms (allele frequency >1%) in the gene.

A total of 10 nucleotide changes were detected in the coding region of the gene plus the flanking intron regions in close proximity of the splice sites (for positions see table 11). Two of these resulted in changes of the amino-acids in the respective codons, as illustrated in table 11.

Two deletions, MUT1 and MUT9, were identified in the untranslated region. MUT1 was detected in the 5'UTR. Two point mutations, MUT2 and MUT3, were also detected in the 5'UTR. These mutations could have an effect on the transcriptional level of the PITX1 RNA. MUT9 was discovered in the 3'UTR and could affect the stability of the RNA.

Two non-synonymous mutations, MUT6 and MUT7, were also found which could have an effect on the function of the protein.

**Table 11**

| ID | Nucleotide position in Seq No ID37 | Alleles | Type of variation | Variation and position in Seq No ID2 | Minor allele frequency |
|---|---|---|---|---|---|
| MUT1 | 632-637 | 632_637delCC GGAG | 5'UTR | | 25% |
| MUT2 | 696 | G/T | 5'UTR | | 15% |
| MUT3 | 823 | A/T | 5'UTR | | 2% |
| MUT4 | 5397 | A/G | intronic | | 31% |
| MUT5 | 5492 | C/A | coding | R140R | 2% |
| MUT6 | 5970 | G/C | coding | G299A | 33% |
| MUT7 | 5973 | T/C | coding | L300P | 2% |
| MUT8 | 6 073 | C/T | 3'UTR | | 9% |
| MUT9 | 6076 | 6076_6081delG CGCGG | 3'UTR | | 30% |
| MUT10 | 6 100 | C/T | 3'UTR | | 3% |

### REFERENCES

Asperger (1944) Die autistischen Psychopathen im Kindesalter. Archiv für Psychiatrie und Nervenkrankheiten, 2:217-250.
Bailey A, Le Couteur A, Gottesman I, et al. (1995) Autism as a strongly genetic disorder: evidence from a British twin study. Psychol Med, 25:63-77.
Bailey A, Phillips W, Rutter M (1996) Autism: towards an integration of clinical, genetic, neuropsychological, and neurobiological perspectives. J Child Psychol Psychiatry 37(1):89-126.
Baird G, Charman T, Baron-Cohen S et al. (2000) A screening instrument for autism at 18 months of age: a 6-year follow-up study. J Am Acad Child Adolesc Psychiatry, 39(6):694-702.
Burger R and Warren R (1998) Possible immunogenetic basis for autism. Ment Retard Dev Disabil Res Rev, 4:137-141.
Carney RM, Wolpert CM, Ravan SA et al. (2003) Identification of MeCP2 mutations in a series of females with autistic disorder. Pediatr Neurol, 28(3):205-211.
Chakrabarti S and Fombonne E (2001) Pervasive developmental disorders in preschool children. JAMA, 285(24):3093-9
Chamberlain RS, Herman BH. (1990) A novel biochemical model linking dysfunctions in brain melatonin, proopiomelanocortin peptides, and serotonin in autism. Biol Psychiatry. 28(9):773-93.
Comi AM, Zimmerman AW, Frye VH et al. (1999) Familial clustering of autoimmune disorders and evaluation of medical risk factors in autism. J Child Neurol, 14(6):388-394.
Connolly AM, Chez MG, Pestronk A et al. (1999) Serum autoantibodies to brain in Landau-Kleffner variant, autism, and other neurologic disorders. J Pediatr, 134(5):607-613.
Curin JM, Terzic J, Petkovic ZB, Zekan L, Terzic IM, Susnjara IM. (2003) Lower cortisol and higher ACTH levels in individuals with autism. J Autism Dev Disord. 33(4):443-8.
Folstein S and Rutter M (1977) Infantile autism: a genetic study of 21 twin pairs. J Child Psychol Psychiatry Allied Disciplines, 18:297-321.
Folstein SE and Rosen-Sheidley BR (2001) Genetics of autism: complex aetiology for a heterogeneous disorder. Nat Rev Genet, 2:943-955.
Gillberg C (1998) Chromosomal disorders and autism. J Autism Dev Disord, 28(5):415-425.
Gillberg C and Coleman M (2000) The biology of the autistic syndromes, 3rd edn London: MacKeith Press.
Gillberg C and Wing L (1999) Autism: not an extremely rare disorder. Acta Psychiatr Scand, 99:339-406.
Hugot JP, Chamaillard M, Zouali H et al. (2001) Association of NOD2 leucine-rich repeat variants with susceptibility to Crohn's disease. Nature 411(6837):599-603.
Jamain S, Quach H, Betancur C et al. (2003) Mutations of the X-linked genes encoding neuroligins NLGN3 and NLGN4 are associated with autism. Nat Genet, 34(1):27-29.
Jones AC, Sampson JR, Hoogendoorn B, Cohen D, Cheadle JP. (2000) Application and evaluation of denaturing HPLC for molecular genetic analysis in tuberous sclerosis. Hum Genet., 106(6):663-8.
Jorde LB, Hasstedt SJ, Ritvo ER et al. (1991) Complex segregation analysis of autism. Am J Hum Genet, 49(5):932-938.
Jorde LB, Mason-Brothers A, Waldmann R et al. (1990) The UCLA-University of Utah epidemiologic survey of autism: genealogical analysis of familial aggregation. Am J Med Genet, 36(1):85-88.
Kanner L (1943) Autistic disturbances of affective contact. Nervous Child, 2:217-250.
Lamonerie, T.; Tremblay, J. J.; Lanctot, C.; Thierrien, M.; Gauthier, Y.; Drouin, J. (1996) Ptx1, a bicoid-related homeo box transcription factor involved in transcription of the pro-opiomelanocortin gene. Genes Dev. 10: 1284-1295.
Lander E and Kruglyak L (1995) Genetic dissection of complex traits: guidelines for interpreting and reporting linkage results. Nat Genet, 11(3):241-247.
Le Couteur A, Rutter M, Lord C et al. (1989) Autism diagnostic interview: a standardized investigator-based instrument. J Autism Dev Disord, 19(3):363-387.
Lesage S, Zouali H, Cezard Jpet al. (2002) CARD15/NOD2 mutational analysis and genotype-phenotype correlation in 612 patients with inflammatory bowel disease. Am J Hum Genet. 70(4):845-857.
Lord C, Rutter M, Le Couteur A (1994) Autism Diagnostic Interview-Revised: a revised version of a diagnostic interview for caregivers of individuals with possible pervasive developmental disorders. J Autism Dev Disord, 24(5):659-685.
Martin, E.R., Monks, S.A., Warren, L.L., and Kaplan, N.L. (2000). A test for linkage and association in general pedigrees: the pedigree disequilibrium test. Am J Hum Genet 67, 146-154.
Nelson KB (1991) Prenatal and perinatal factors in the etiology of autism. Pediatrics, 87(5 Pt 2):761-766.
Pellegrini-Bouiller, I.; Manrique, C.; Gunz, G.; Grino, M.; Zamora, A. J.; Figarella-Branger, D.; Grisoli, F.; Jaquet, P.; Enjalbert, A. (1999) Expression of the members of the Ptx family of transcription factors in human pituitary adenomas. J. Clin. Endocr. Metab. 84: 2212-2220.
Rioux JD, Daly MJ, Silverberg MS et al. (2001) Genetic variation in the 5q31 cytokine gene cluster confers susceptibility to Crohn disease. Nat Genet 29(2): 223-228.
Rioux JD, Silverberg MS, Daly MJ (2000) Genomewide search in Canadian families with inflammatory bowel disease reveals two novel susceptibility loci. Am J Hum Genet 66(6):1863-1870.
Rodier P and Hyman S (1998) Early environmental factors in autism. Mental Retard Dev Disord Res Rev, 4:121-128.
Shapiro, M. D.; Marks, M. E.; Peichel, C. L.; Blackman, B. K.; Nereng, K. S.; Jonsson, B.; Schluter, D.; Kingsley, D. M.(2004) Genetic and developmental basis of evolutionary pelvic reduction in threespine sticklebacks. Nature 428: 717-723.
Singh VK, Warren RP, Odell JD et al. (1993) Antibodies to myelin basic protein in children with autistic behavior. Brain Behav Immun, 7(1):97-103.
Smalley SL (1997) Genetic influences in childhood-onset psychiatric disorders: autism and attention-deficit/hyperactivity disorder. Am J Hum Genet, 60(6):1276-1282.
Steffenburg S, Gillberg C, Hellgren L et al. (1989) A twin study of autism in Denmark, Finland, Iceland, Norway and Sweden. J Child Psychol Psychiatry, 30(3):405-416:
Szatmari P, Jones MB, Zwaigenbaum L et al. (1998) Genetics of autism: overview and new directions. J Autism Dev Disord, 28(5):351-368.
Szeto, D. P.; Rodriguez-Esteban, C.; Ryan, A. K.; O'Connell, S. M.; Liu, F.; Kioussi, C.; Gleiberman, A. S.; Izpisua-Belmonte, J. C.; Rosenfeld, M. G. (1999) Role of the Bicoid-related homeodomain factor Pitx1 in specifying hindlimb morphogenesis and pituitary development. Genes Dev. 13: 484-494.
Tremblay JJ, Lanctot C, Drouin J. (1998) The pan-pituitary activator of transcription, Ptx1 (pituitary homeobox 1), acts in synergy with SF-1 and Pit1 and is an upstream regulator of the Lim-homeodomain gene Lim3/Lhx3. Mol Endocrinol. 12(3):428-41.
Weizman A, Weizman R, Szekely GA et al. (1982) Abnormal immune response to brain tissue antigen in the syndrome of autism. Am J Psychiatry, 139(11):1462-1465.

## Claims

1. An in vitro method of detecting the presence of or predisposition to autism, or to an autism spectrum disorder in a subject, the method comprising detecting the presence of an alteration in the PITX1 gene locus in a sample from the subject.

2. An in vitro method of detecting the protection from autism, or from an autism spectrum disorder, the method comprising detecting the presence of an alteration in the PITX1 gene locus in a sample from the subject.

3. An in vitro method of assessing the response of a subject to a treatment of autism or to an autism spectrum disorder, the method comprising detecting the presence of an alteration in the PITX1 gene locus in a sample from the subject.

4. An in vitro method of assessing the adverse effect in a subject to a treatment of autism, or of an autism spectrum disorder, the method comprising detecting the presence of an alteration in the PITX1 gene locus in a sample from the subject.

5. The method of any one of claims 1-4, wherein the presence of an alteration in the PITX1 gene locus is detected by sequencing, selective hybridisation and/or selective amplification.

6. The method of any one of claims 1-4, wherein said alteration is one or several SNP(s) or a haplotype of SNPs associated with autism.

7. The method of claim 6, wherein said SNPs are selected from the group consisting of those disclosed in Tables 1a and 1b, more preferably those disclosed in Tables 3-8.

8. The method of any one of claims 6-7, wherein said SNP(s) associated with autism are selected from the group consisting of SNP6 and SNP33.

9. The method of any one of claims 6-7, wherein said haplotype associated with autism comprises or consists of several SNPs selected from the group consisting of SNP6, SNP33, SNP25, SNP27, SNP29, SNP31 and SNP33.

10. The method of claim 9, wherein said haplotype consists of or comprises SNP24, SNP25 and SNP40, preferably with the alleles C-T-A.

11. The method of claim 9, wherein said haplotype consists of or comprises SNP23, SNP25 and SNP33, preferably with the alleles A-T-G.

12. The method of claim 9, wherein said haplotype consists of or comprises SNP25, SNP27, SNP29 and SNP31, preferably with the alleles T-A-C-A.

13. The method of any one of claims 6-7, wherein said haplotype associated with autism is selected from the haplotypes disclosed in Tables 4, 6, 7 and/or 8.

14. An in vitro method of selecting biologically active compounds on autism, or on autism spectrum disorders, said method comprising contacting a test compound with a PITX1 polypeptide or gene or a fragment thereof and determining the ability of said test compound to bind the PITX1 polypeptide or gene or a fragment thereof.

15. An in vitro method of selecting biologically active compounds on autism, or on autism spectrum disorders, said method comprising contacting a recombinant host cell expressing a PITX1 polypeptide with a test compound, and determining the ability of said test compound to bind said PITX1 polypeptide and to modulate the activity of PITX1 polypeptide.

16. An in vitro method of selecting biologically active compounds on autism, or on autism spectrum disorders, said method comprising contacting a test compound with a PITX1 gene and determining the ability of said test compound to modulate the expression of said PITX1 gene.

17. A in vitro method of selecting biologically active compounds on autism, or on autism spectrum disorders, said method comprising contacting a test compound with a recombinant host cell comprising a reporter construct, said reporter construct comprising a reporter gene under the control of a PITX1 gene promoter, and selecting the test compounds that modulate (e.g. stimulate or reduce) expression of the reporter gene.

18. Method according any one of claims 14-17, wherein said PITX1 gene or polypeptide or a fragment thereof is an altered or mutated PITX1 gene or polypeptide or a fragment thereof comprising the alteration or mutation.

19. Method according any one of claims 14-17, wherein said modulation is an activation.

20. Method according any one of claims 14-17, wherein said modulation is an inhibition.

## Patentansprüche

1. Ein *in vitro* Verfahren zum Nachweis des Vorhandenseins oder der Veranlagung zu Autismus oder zu einer Störung des autistischen Spektrums bei einem Patienten, wobei das Verfahren den Nachweis des Vorhandenseins einer Veränderung in dem PITX1 Genlocus in einer Probe von dem Patienten umfasst.

2. Ein *in vitro* Verfahren zum Nachweis des Schutzes vor Autismus oder vor einer Störung des autistischen Spektrums, wobei das Verfahren den Nachweis des Vorhandenseins einer Veränderung in dem PITX1 Genlocus in einer Probe von dem Patienten umfasst.

3. Ein *in vitro* Verfahren zur Bewertung des Ansprechens von einem Patienten auf eine Behandlung von Autismus oder einer Störung des autistischen Spektrums, wobei das Verfahren den Nachweis des Vorhandenseins einer Veränderung in dem PITX1 Genlocus in einer Probe von dem Patienten umfasst.

4. Ein *in vitro* Verfahren zur Bewertung der Nebenwirkung bei einem Patienten auf die Behandlung von Autismus oder einer Störung des autistischen Spektrums, wobei das Verfahren den Nachweis des Vorhandenseins einer Veränderung in dem PITX1 Genlocus in einer Probe von dem Patienten umfasst.

5. Das Verfahren nach einem der Ansprüche 1 bis 4, wobei das Vorhandensein von einer Veränderung in dem PITX1 Genlocus durch Sequenzierung, selektive Hybridisierung und/oder selektive Amplifikation nachgewiesen wird.

6. Das Verfahren nach einem der Ansprüche 1 bis 4, wobei die Veränderung ein oder mehrere SNP(s) oder ein mit Autismus assozierter Haplotyp von SNPs ist.

7. Das Verfahren nach Anspruch 6, wobei diese SNPs ausgewählt sind aus der Gruppe bestehend aus solchen, die in Tabellen 1a und 1b aufgelistet sind, im Speziellen solche, die in den Tabellen 3 bis 8 aufgelistet sind.

8. Das Verfahren nach einem der Ansprüche 6 bis 7, wobei diese mit Autismus assoziierten SNP(s) ausgewählt sind aus der Gruppe bestehend aus SNP6 und SNP33.

9. Das Verfahren nach einem der Ansprüche 6 bis 7, wobei dieser mit Autsimus assoziierte Haplotyp mehrere SNPs, ausgewählt aus der Gruppe bestehend aus SNP6, SNP33, SNP25, SNP27, SNP29, SNP31 und SNP33, umfasst oder aus diesen besteht.

10. Das Verfahren nach Anspruch 9, wobei dieser Haplotyp SNP24, SNP25 und SNP40, vorzugsweise mit den Allelen C-T-A, umfasst oder aus diesen besteht.

11. Das Verfahren nach Anspruch 9, wobei der Haplotyp SNP23, SNP25 und SNP33, vorzugsweise mit den Allelen A-T-G, umfasst oder aus diesen besteht.

12. Das Verfahren nach Anspruch 9, wobei der Haplotyp SNP25, SNP27, SNP29 und SNP31, vorzugsweise mit den Allelen T-A-C-A, umfasst oder aus diesen besteht.

13. Das Verfahren nach einem der Ansprüche 6 bis 7, wobei der mit Autismus assozierte Haplotyp ausgewählt ist von den Haplotypen, die in Tabellen 4, 6, 7 und/oder 8 aufgelistet sind.

14. Ein *in vitro* Verfahren zur Auswahl bzgl. Autismus oder Störungen des autistischen Spektrums biologisch aktiver Verbindungen, wobei dieses Verfahren das in Kontakt bringen einer Testverbindung mit einem PITX1-Polypetid oder -Gen oder ein Fragment davon und die Untersuchung des Vermögens dieser Testverbindung das PITX1-Polypeptid oder -Gen oder ein Fragment davon zu binden umfasst.

15. Ein *in vitro* Verfahren zur Auswahl bzgl. Autismus oder Störungen des autistischen Spektrums biologisch aktiver Verbindungen, wobei dieses Verfahren das in Kontakt bringen einer rekombinanten Wirtszelle, die ein PITX1-Polypeptid exprimiert, mit einer Testverbindung und die Untersuchung des Vermögens dieser Testverbindung das PITX1-Polypeptid zu binden und die Aktivität des PITX1-Polypeptids zu modulieren umfasst.

16. Ein *in vitro* Verfahren zur Auswahl bzgl. Autismus oder Störungen des autistischen Spektrums biologisch aktiver Verbindungen, wobei diese Methode das in Kontakt bringen einer Testverbindung mit einem PITX1-Gen und die Untersuchung des Vermögens der Testverbindung die Expression des PITX1-Gens zu modulieren umfasst.

17. Ein *in vitro* Verfahren zur Auswahl bzgl. Autismus oder Störungen des autistischen Spektrums biologisch aktiver Verbindungen, wobei das Verfahren das in Kontakt bringen einer Testverbindung mit einer ein Reporterkonstrukt umfassende rekombinanten Wirtszelle, wobei dieses Reporterkonstrukt ein Reportergen unter der Kontrolle von einem PITX1-Genpromotor umfasst, und Auswahl der Testverbindungen, die die Expression des Reportergens modulieren (zum Beispiel stimulieren oder reduzieren) umfasst.

18. Das Verfahren nach einem der Ansprüche 14 bis 17, wobei das PITX1-Gen oder -Polypeptid oder Fragment davon ein geändertes oder mutiertes PITX1-Gen oder -Polypeptid oder Fragment davon ist, das eine Änderung oder Mutation umfasst.

19. Das Verfahren nach einem der Ansprüche 14 bis 17, wobei die Modulation eine Aktivierung ist.

20. Das Verfahren nach einem der Ansprüche 14 bis 17, wobei die Modulation eine Inhibierung ist.

## Revendications

1. Procédé in vitro de détection de la présence ou d'une prédisposition à l'autisme, ou à un désordre du spectre de l'autisme, chez un sujet, le procédé comprenant la détection de la présence d'une altération dans le locus du gène PITX1 dans un échantillon du sujet.

2. Procédé in vitro de détection de la protection envers l'autisme, ou envers un désordre du spectre de l'autisme, le procédé comprenant la détection de la présence d'une altération dans le locus du gène PITX1 dans un échantillon du sujet.

3. Procédé in vitro d'évaluation de la réponse d'un sujet à un traitement de l'autisme, ou à un désordre du spectre de l'autisme, le procédé comprenant la détection de la présence d'une altération dans le locus du gène PITX1 dans un échantillon du sujet.

4. Procédé in vitro d'évaluation de l'effet secondaire chez un sujet vis à vis d'un traitement de l'autisme, ou d'un désordre du spectre de l'autisme, le procédé comprenant la détection de la présence d'une altération dans le locus du gène PITX1 dans un échantillon du sujet.

5. Procédé selon l'une quelconque des revendications 1-4, dans lequel la présence d'une altération dans le locus du gène PITX1 est détectée par séquençage, hybridation sélective et/ou amplification sélective.

6. Procédé selon l'une quelconque des revendications 1-4, dans lequel ladite altération est un ou plusieurs SNP(s) ou un haplotype de SNPs associés à l'autisme.

7. Procédé selon 1a revendication 6, dans lequel lesdits SNPs sont choisis dans le groupe consistant en ceux décrits dans les Tableaux 1a et 1b, plus préférentiellement ceux décrits dans les Tableaux 3-8.

8. Procédé selon l'une quelconque des revendications 6-7, dans lequel le(s)dit(s) SNP(s) associé(s) à l'autisme sont choisis dans le groupe consistant en SNP6 et SNP33.

9. Procédé selon l'une quelconque des revendications 6-7, dans lequel ledit haplotype associé à l'autisme comprend ou consiste en plusieurs SNPs choisis dans le groupe consistant en SNP6, SNP33, SNP25, SNP27, SNP29, SNP31 et SNP33.

10. Procédé selon la revendication 9, dans lequel ledit haplotype consiste en ou comprend SNP24, SNP25 et SNP40, de préférence avec les allèles C-T-A.

11. Procédé selon la revendication 9, dans lequel ledit haplotype consiste en ou comprend SNP23, SNP25 et SNP33, de préférence avec les allèles A-T-G.

12. Procédé selon la revendication 9, dans lequel ledit haplotype consiste en ou comprend SNP25, SNP27, SNP29 et SNP31, de préférence avec les allèles T-A-C-A.

13. Procédé selon l'une quelconque des revendications 6-7, dans lequel ledit haplotype associé à l'autisme est choisi parmi les haplotypes décrits dans les Tableaux 4, 6, 7 et/ou 8.

14. Procédé in vitro de sélection de composés biologiquement actifs sur l'autisme, ou sur des désordres du spectre de l'autisme, ledit procédé comprenant la mise en contact d'un composé test avec un gène ou un polypeptide PITX1 ou un fragment de celui-ci, et la détermination de la capacité dudit composé test à se lier au gène ou au polypeptide PITX1 ou au fragment de celui-ci.

15. Procédé in vitro de sélection de composés biologiquement actifs sur l'autisme, ou sur des désordres du spectre de l'autisme, ledit procédé comprenant la mise en contact d'une cellule hôte recombinante exprimant un polypeptide PITX1 avec un composé test, et la détermination de la capacité dudit composé test à se lier au polypeptide PITX1 et à moduler l'activité du polypeptide PITX1.

16. Procédé in vitro de sélection de composés biologiquement actifs sur l'autisme, ou sur des désordres du spectre de l'autisme, ledit procédé comprenant la mise en contact d'un composé test avec un gène PITX1, et la détermination de la capacité dudit composé test à moduler l'expression dudit gène PITX1.

17. Procédé in vitro de sélection de composés biologiquement actifs sur l'autisme, ou sur des désordres du spectre de l'autisme, ledit procédé comprenant la mise en contact d'une cellule hôte recombinante comprenant un construit rapporteur, ledit construit rapporteur comprenant un gène rapporteur sous le contrôle d'un promoteur du gène PITX1, et la sélection des composés tests qui modulent (par exemple qui stimulent ou réduisent) l'expression du gène rapporteur.

18. Procédé selon l'une quelconque des revendications 14-17, dans lequel ledit gène ou polypeptide PITX1 ou un fragment de celui-ci est un gène ou un polypeptide PITX1 altéré ou muté ou un fragment de celui-ci comprenant l'altération ou la mutation.

19. Procédé selon l'une quelconque des revendications 14-17, dans lequel ladite modulation est une activation.

20. Procédé selon l'une quelconque des revendications 14-17, dans lequel ladite modulation est une inhibition.
